# EUROPEAN PATENT SPECIFICATION

(11) **EP 1 073 661 B1**
(45) Date of publication and mention of the grant of the patent: **02.03.2005**
(21) Application number: 99911971.2
(22) Date of filing: 15.04.1999
(51) Int. Cl.: C07D 498/04, C09B 62/04, C09B 19/02

(54) **DIOXAZINE DERIVATIVES AND THEIR USE AS DYESTUFFS**
DIOXAZIN-DERIVATE UND IHRE VERWENDUNG ALS FARBSTOFFE
DERIVES DE DIOXAZINE ET LEUR UTILISATION COMME COLORANTS

(30) Priority: 18.04.1998 GB 9808167; 27.04.1998 GB 9808778
(43) Date of publication of application: 07.02.2001
(73) Proprietor: Clariant Finance (BVI) Limited, Road Town, Tortola (VG)
(72) Inventor: HASEMANN, Ludwig, D-79379 Müllheim-Niederweiler (DE)
(74) Representative: D'haemer, Jan Constant
(86) International application number: PCT/IB1999/000659
(87) International publication number: WO 1999/054334

(56) References cited:
- EP-A- 0 373 534
- EP-A- 0 485 329
- EP-A- 0 773 264
- WO-A-93/18224
- US-A- 5 122 605
- US-A- 5 585 489
- US-A- 5 653 773

## Description

The invention relates to dioxazine compounds containing sulphonic acid groups and salts thereof and mixtures of these compounds which may be in internal or external salt form. They are suitable for use as dyestuffs.

US5122605 discloses a sulpho group-containing dioxazine compounds useful for dyeing or printing hydroxy group- or nitrogen-containing organic substrates, optionally combined with a special after-treatment of the dyed or printed textiles thus obtained to improve their wet fastness properties.

US5653773 discloses a process for dyeing or printing fibre materials containing hydroxyl groups wherein at least one blue-dyeing dioxazine compound is used together with at least one yellow-dyeing and/or at least one red-dyeing and/or at least one navy blue- or black-dyeing dyestuff.

According to the invention there are provided compounds of formula (I) 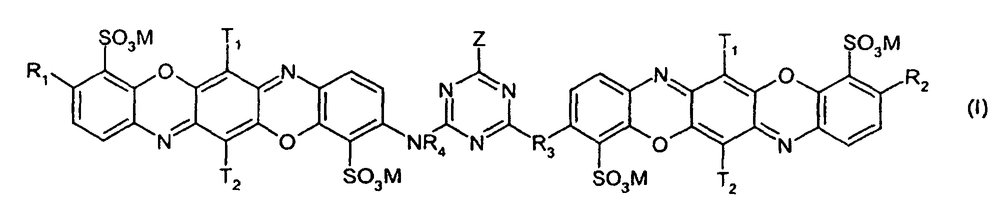 wherein
- R₁: signifies -NH₂ or 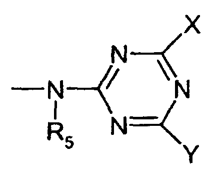 wherein
R₅ signifies hydrogen or C₁₋₄-alkyl,
X and Y independently signify halogen or hydroxy or C₁₋₃-alkoxy or phenoxy or the radical of a cyclic, an aliphatic, an araliphatic or an aromatic amine linked over the amine-nitrogen and optionally substituted by hydroxy, carboxy, alkoxy, alkyl and/or sulphonic acid groups or the rest of a heterocyclic amine linked over the amine-nitrogen,
- Z: signifies a radical of the formula 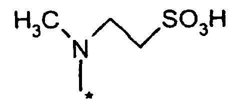 wherein the aterix show the point of attachement,
- R₂: signifies -NH₂ or 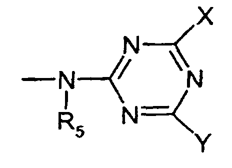 with the same definitions for R₅, X and Y as above or
- R₂: is the rest (b) 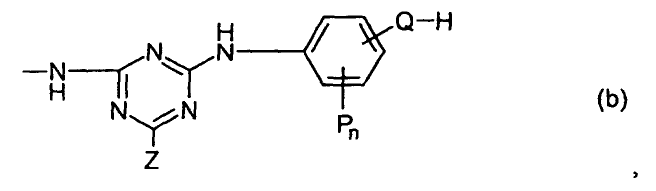 wherein
P signifies -SO₃H, -COOH or -OH,
Q signifies a -NH- radical or a group selected from 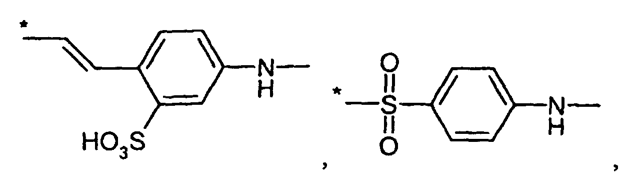 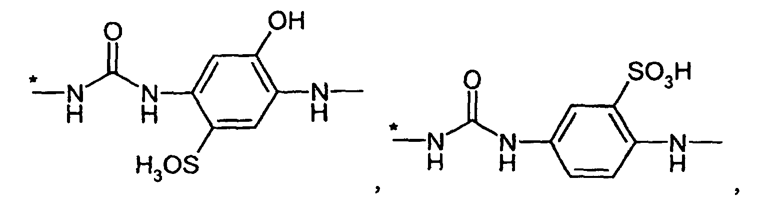 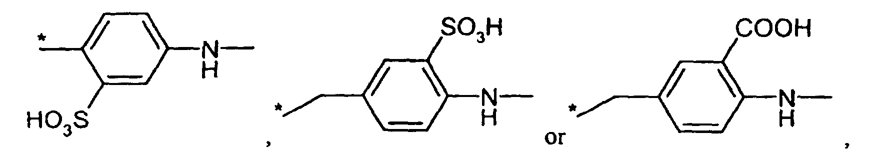 wherein the asterisk marks the bond attached to the phenyl ring,
Z signifies the same as above,
n has the value 0, 1, 2,
- R₃: signifies -NR₆
in which R₆ signifies hydrogen or a C₁₋₄-alkyl radical
or
- R₃: is the rest (a) 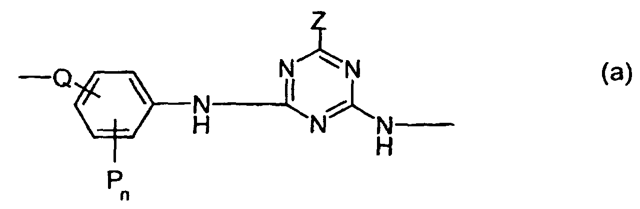
wherein all substituents have the same meanings as defined above,
- R₄: signifies hydrogen or C₁₋₄-alkyl,
- T₁ and T₂: independently from each other signify hydrogen, halogen, C₁₋₆-alkyl or C₁₋₄-alkoxy,
- M: signifies hydrogen or a cation;
salts thereof and mixtures of such compounds and/or salts.

Preferred compounds of formula (I), wherein all substituents have same meanings as defined above, have the provisos that
(i) when R₁ and R₂ is -NH₂ then R₃ is 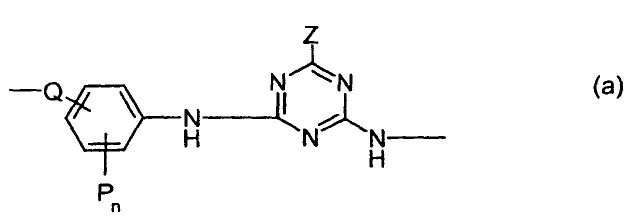 wherein P, Q, Z and n have the same meanings as defined above,
(ii) when R₁ is -NH₂ and R₂ is 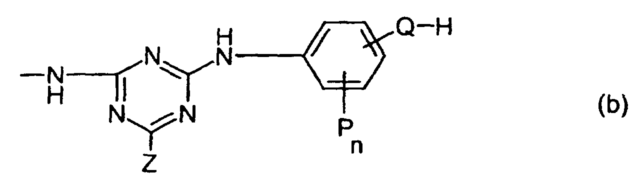 wherein P, Q, Z and n have the same meaning as defined above,
   then R₃ is -NH-;
salts thereof and mixtures of such compounds and/or salts.

In further preferred compounds of formula (I) both R₁ and R₂ signify 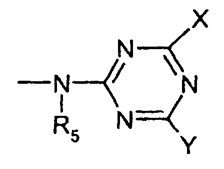 wherein
- R₅: signifies hydrogen or C₁₋₄-alkyl,
and
- R₃: signifies -NR₆
in which R₆ signifies hydrogen or a C₁₋₄-alkyl radical and
all the other substituents have the definition as defined above,
salts thereof and mixtures of such compounds and/or salts.

In further preferred compounds the radicals X, Y and Z contain no chromophoric group.

In other preferred compounds any amine H-X, H-Y or H-Z has a molecular weight in the range of 50 to 500, preferably 50 to 400. Where such an amine contains ring systems, it preferably contains 1 to 4 rings and most preferably has only 1 or 2 rings. Such amine preferably contains at least one hydrophilic group which independently can be anionic, cationic or non-ionogenic. Examples of anionic hydrophilic groups are carboxy or sulphonic acid groups. Examples of cationic hydrophilic groups are mono-(C₁₋₄-alkyl)- or di(C₁₋₄-alkyl)amino groups comprising a protonatable nitrogen atom or a quaternary ammonium group, wherein each C₁₋₄-alkyl group can be substituted by halogen, hydroxy, C₁₋₄-alkoxy, phenyl or phenoxy. Any phenyl or naphthyl ring present in the amine may be unsubstituted or substituted by one, two or three groups selected from halogen, hydroxy, C₁₋₄-alkyl, C₁₋₄-alkoxy, phenoxy, carboxy or sulphonic acid. Any heterocyclic ring present in the amine is a 5-or 6-membered ring containing one or two hetero atoms selected from N, O or S, which heterocyclic ring is unsubstituted or substituted by one or two C₁₋₄-alkyl groups.

Preferred H-Z are ammonia and aliphatic amines, preferably substituted with a hydroxy, carboxy, alkoxy or sulphonic acid group, e.g. ethanolamine, diethanolamine, isopropanolamine, diisopropanolamine, 2-amino-hydroxypropane, glycine, N-methylethanolamine, 3-methoxy-propylamine, 1-aminoethyl-2-sulfonic acid and most preferably, 1-methylamino-ethyl-2-sulfonic acid; heterocyclic amines, e.g. morpholine, piperazine or hydroxy-ethylpiperazine; or N,N-diethylaminopropylamine and 1,2-diaminopropane.

Most preferred H-Z' is 1-methylamino-ethyl-2-sulfonic acid.

In a further preferred compound or mixture M is hydrogen or a colorless cation or M is a cationic portion in a substituent X, Y or Z containing a cationic charge, to form an inner salt.

In a further aspect of the present invention there are provided mixtures containing
a) a compound of formula (Ia) 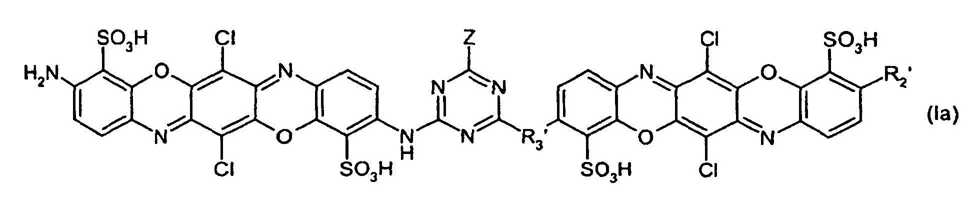 wherein R₃' is a divalent radical of formula (a), R₂' is -NH₂ and Z has the same meaning as defined above;
b) a compound of the above formula (Ia) wherein R₃' is -NH-, R₂' is a radical of formula (b) and Z has the same meaning as defined above;
c) a compound of formula (II) 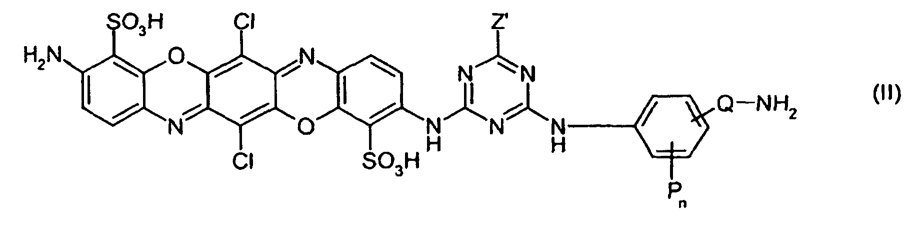 wherein Q, P and n have the same meaning as defined above and Z' has the same definition as Z, additionally Z' can be an aromatic amine, preferably for the component c) Z' is a radical of formula (c) 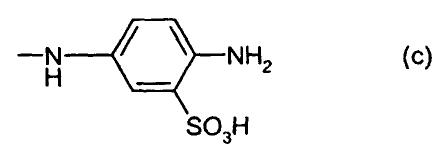
d) a compound of formula (II), wherein Z has the same meanings as defined above, except halogen, preferably for the component d) Z' is a radical of formula (d) 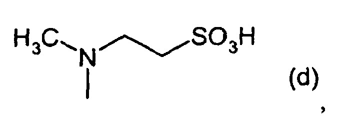
e) a compound of formula (III) 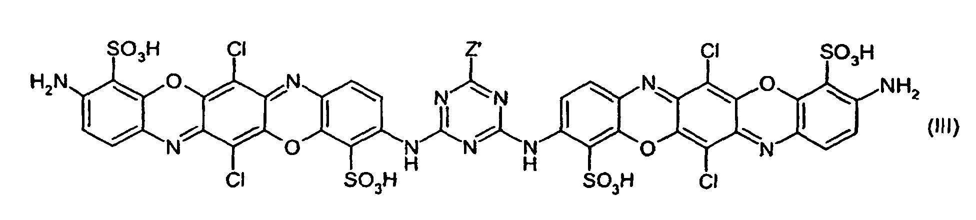 wherein Z' has the definition as defined above,
or salts of such compounds.

The present invention further provides a process for the preparation of a compound of formula (Ib) 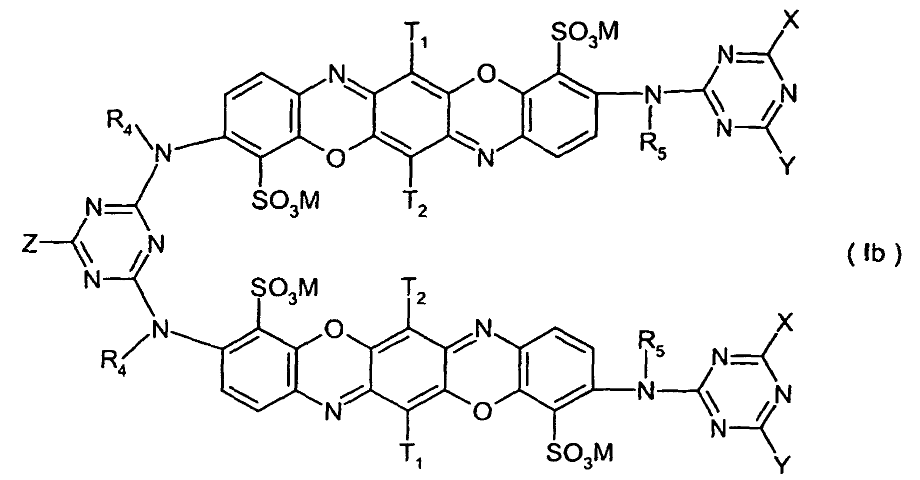 according to formula (I) with the same definitions of the substituents as defined above,
wherein one mole of a compound of formula (IV) 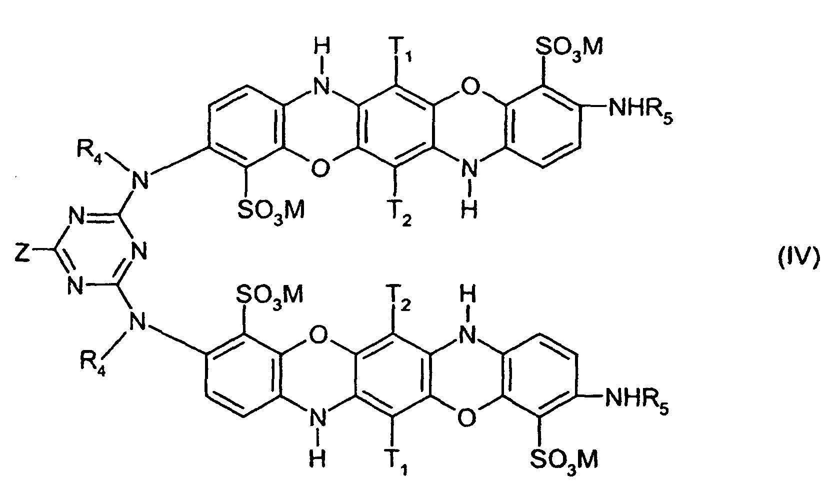 or a mixture of compounds of formula (IV) with the same definitions of the substituents as in formula (Ib) is reacted with two moles of at least one compound of formula (V) 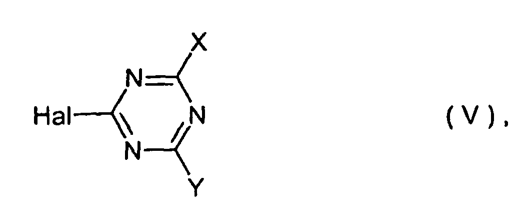 wherein Hal signifies halogen, preferably chlorine, and X and Y independently have the same meanings as defined above, under dehydrohatogenating conditions, and, if in formula (Ib) anyone of X and Y has a significance other than halogen while in formula (V) the corresponding X or Y signifies halogen, and/or if in formula (Ib) Z has a significance other than halogen, the obtained condensation product of formula (VI) 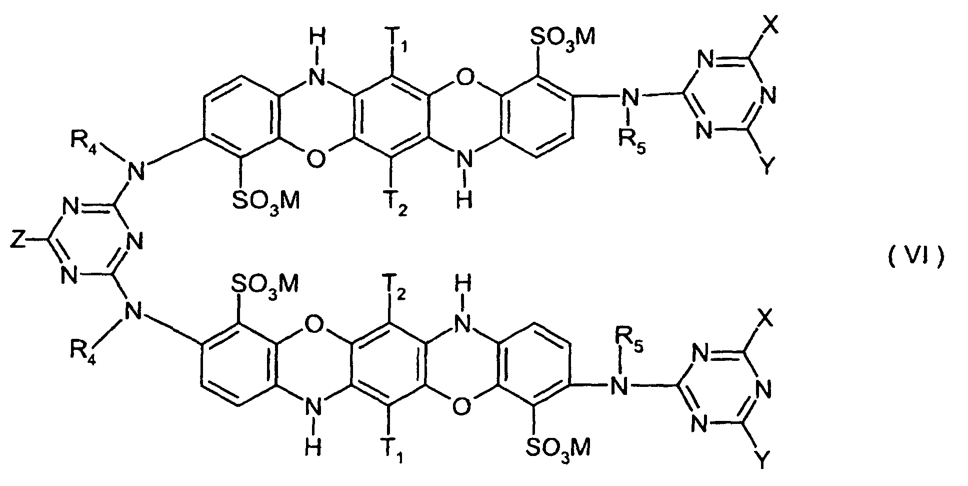 with the same definitions of the substituents as in formula (Ib) is reacted, with at least one corresponding amine of formula H-X, H-Y and/or H-Z, which have the same definitions as above and/or an alkali metal C₁₋₃-alcoholate or phenolate, as required. The oxidation of the leuco form to the dioxazine form takes place after the condensation step(s) or simultaneously with the exchange of halogen for amine.

The compounds of formula (Ib) thus obtained may be isolated in accordance with known methods.

The compounds of formula (Ib) containing free basic groups may be converted wholly or in part into water-soluble salts by reacting with any inorganic or organic acids. The compounds of formula (Ib) containing carboxy or sulphonic acid groups may also be converted into water-soluble salts by reacting with any basic compound.

The starting compounds, the amines of formula (IV) in the leuco form may be prepared by step-wise replacement of the chlorine atoms of cyanuric chloride whereby in a first and second step cyanuric chloride is reacted with a dioxazine compound of formula (VII) 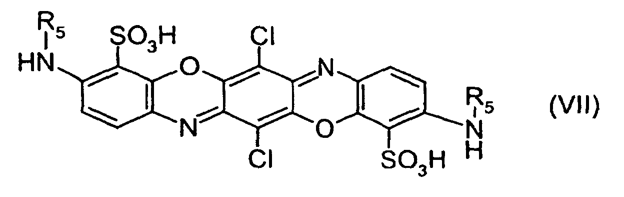 which can be identical or different, depending on the meanings of R₅.

Before the reaction with a compound of the formula (V), the intermediately obtained dyestuff is brought into the leuco form of formula (IV) by reduction. This reduction process can be performed catalytically with hydrogen (and the usual catalysts palladium, platinum or nickel) or with other metals like iron, tin or zinc in the presence of acids. Alternatively, the reduction can be performed with sodium dithionite in water at pH values from 6 to 9, preferably 6.5 to 7.5, and temperatures from 15 to 45°C, preferably 20 to 30°C. The reducing agent is suitably used in double or triple molar amounts of the dyestuff to be reduced and all reduction and condensation reactions are performed in inert atmosphere, e.g. under nitrogen.

The compounds of formula (V) may also be prepared by step-wise replacement of the chlorine atoms of cyanuric chloride whereby in a first and/or second step, cyanuric chloride is reacted with an amine of formula HX and/or an amine of formula HY.

In the case where identical amino groups have to be introduced, this first and second steps may be combined into one step. Suitably, the single step is carried out at temperatures from 0 - 30°C and preferably at pH 4 - 6.

Where different amino groups have to be introduced, suitably, the amine showing the higher selectivity with respect to the condensation reaction is introduced in the first step at a temperature of preferably 0 - 20°C more preferably 0 - 5°C. Both condensation steps may be carried out using the conventional reaction medium where the upper limit of pH is 7. The second step is preferably carried out at 10 - 40°C, more preferably 12 - 30°C.

The starting compounds of formulae (VII), HX and HY are either known or may be prepared in accordance with known methods from available starting materials.
The present invention further provides a process for the preparation of compounds of the formula (Ia), 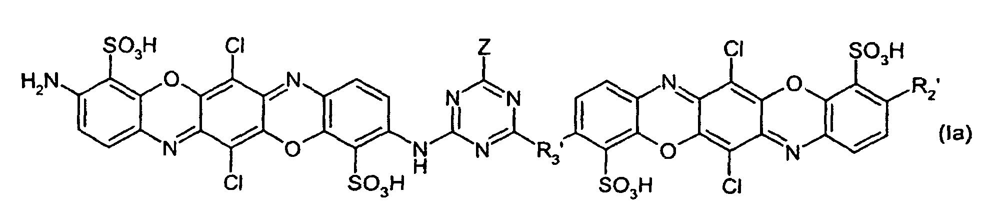 according to formula I wherein either
R₃' is a divalent radical of formula (a) 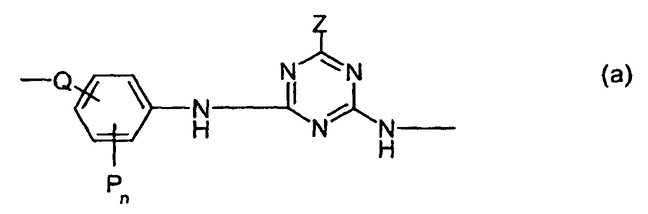 wherein P, Q, Z and n have the same meanings as defined above
and R₂' is -NH₂;
or
R₃' is -NH- and
R₂' is a radical of formula (b) 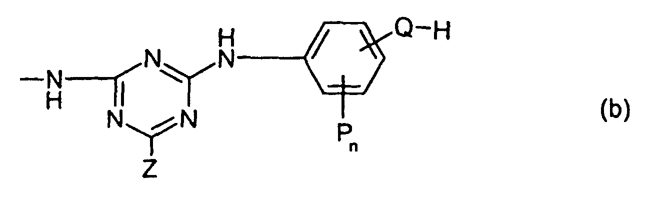 wherein P, Q, Z and n have the same meanings as defined above,
salts thereof and of mixtures of such compounds and/or salts, which comprises reacting a compound of formula (VIII) 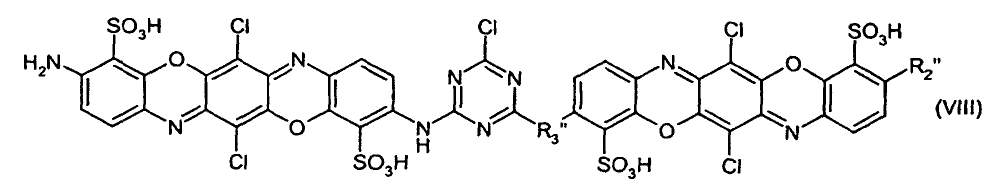 wherein either R₃" is a divalent radical of formula (a') 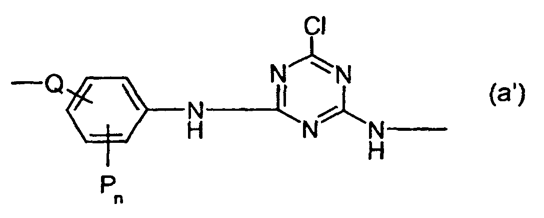 wherein P, Q and n have the same meanings as defined above
and R₂" is -NH₂; or R₃" is -NH- and R₂" is a radical of formula (b') 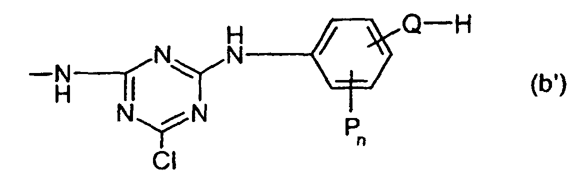 wherein P, Q and n have the same meanings as defined above or a mixture of such compounds with an amine H-Z or H-Z' and, if desired, converting compounds of formula (I) thus obtained into salts or salts thus obtained into compounds of formula (I) or into other salts.

The compounds of the above formula (VIII) can be prepared by reacting a compound of formula (IX) 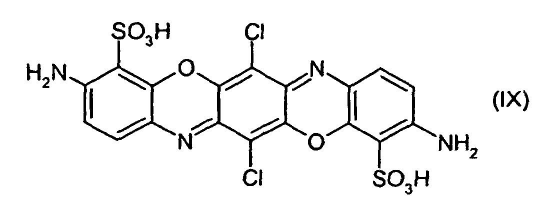 with about one equivalent of 2,4,6-trichlorotriazine, reacting the product of formula (X) 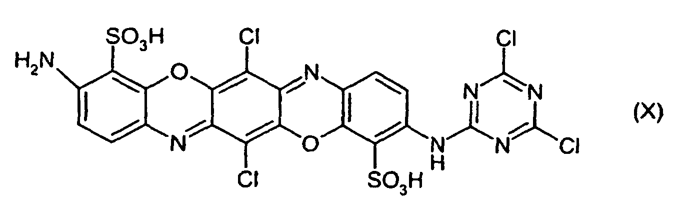 thus obtained with 1,4-diaminobenzene-2-sulfonic acid and reacting the product (XI) 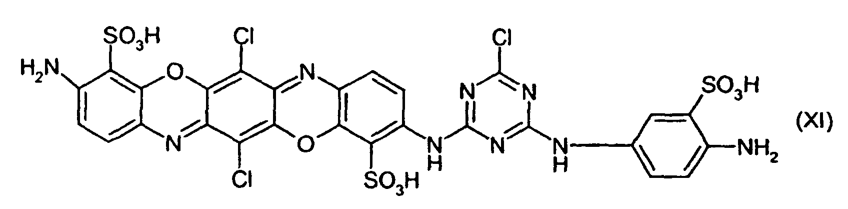 thus obtained with another portion of the compound of the above formula (X).

The compound of the above formula (X) can react (rather than with the 1,4-diaminobenzene-2-sulfonic acid) with any unreacted compound of the above formula (IX) and thus give a compound corresponding to the above formula (III) but in which Z' is chloro which, in the ultimate treatment with an amine H-Z', gives a compound of the above formula (III) in which Z' is a radical as defined above. Similarly, the compound (XI) obtained when reacting a compound of the above formula (X) with 1,4-diaminobenzene-2-sulfonic acid can react (rather than with another portion of the compound of formula (X)) with any unreacted 1,4-diaminobenzene-2-sulfonic acid to give a compound of the above formula (II) wherein Z a radical of the above formula (c), or in the ultimate treatment with an amine H-Z give a compound of the above formula (II) wherein Z is a radical of formula (d) or a radical as defined above. Thus, when
a) reacting a compound of the above formula (IX) with about one equivalent of 2,4,6-trichlorotriazine;
b) reacting the compound of the above formula (X) thus obtained with 1,4-diaminobenzene-2-sulfonic acid;
c) reacting the compound of the above formula (XI) thus obtained with another portion of the compound of the above formula (X); and
d) reacting the product thus obtained with an amine;
there is obtained a mixture as defined hereinabove comprising
a) a compound of the above formula (Ia) wherein R₃' is a divalent radical of the above formula (a) and R₂' is -NH₂ and Z as defined above;
b) a compound of the above formula (Ia) wherein R₃' is -NH- and R₂' is a radical of the above formula (b) and Z as defined above;
c) a compound of the above formula (II) wherein Z' is a radical of the above formula (c) and all the other substituents have the same meanings as defined above;
d) a compound of the above formula (II) wherein Z' is a radical of an amine, preferably a radical of formula (d) and all the other substituents have the same meanings as defined above and
e) a compound of the above formula (III) wherein Z' is a radical of an amine,
or salts of these compounds.

If desired, a mixture of compounds thus obtained can be converted into a mixture of corresponding salts and, similarly, a mixture of salts thus obtained can be converted into a mixture of the corresponding compounds or into a mixture of other salts.

It will be appreciated that the relative quantities of the components of the mixture will depend on, inter alia, the exact reaction conditions used when preparing this mixture. In general, however, these relative quantities are approximately as follows:
Component a): 30 - 40 %;
Component b): 30 - 47 %;
Component c): 1.5 - 8 %;
Component d): 2 - 11 %; and
Component e): 12.5 - 23.5 %.

Suitable amines H-Z' are ammonia and aliphatic amines, preferably substituted with a hydroxy, carboxy, alkoxy and/or sulphonic acid group, e.g. ethanolamine, diethanolamine, isopropanolamine, diisopropanolamine, 2-amino-hydroxypropane, glycine, N-methylethanolamine, 3-methoxy-propylamine, 1-aminoethyl-2-sulfonic acid and most preferably, 1-methylamino-ethyl-2-sulfonic acid; aromatic amines, preferably substituted with a carboxy or sulphonic acid group, e.g. aniline, 4- or 3-sulpho-aniline, 4- or 3-carboxy-aniline; and heterocyclic amines, e.g. morpholine, piperazine or hydroxyethylpiperazine; or N,N-diethylaminopropylamine and 1,2-diaminopropane.

Suitable amines H-Z are the same as for H-Z' except the aromatic amines, with the proviso, that very reactive aromatic amines are suitable.

The compound of formula (IX), 3,10-diamino-6,13-dichloro-4,11-triphendioxazine disulphonic acid, is known. For the reaction with the 2,4,6-trichlorotriazine, the compound of formula (IX) is conveniently taken up in water, and the pH is brought to about 8.0 - 8.5 by addition of caustic alkali, preferably an aqueous lithium hydroxide solution. The 2,4,6-trichlorotriazine is conveniently utilized in the form of an aqueous suspension containing a small amount of a conventional surfactant, which is slowly added, under cooling to a temperature of about 0 - 10°C, preferably about 5 - 8°C, to the aqueous solution of the compound of formula (IX) under stirring, the pH of the reaction mixture being kept at about 8.0 - 8.5 by continuous addition of further caustic alkali, preferably aqueous lithium hydroxide solution, and the temperature being kept at about 0 - 10°C, preferably at about 5 - 8°C. Normally, the reaction is finished after about one hour. There is thus obtained an aqueous suspension of the compound of formula (X).

Conveniently about one half of the above suspension of the compound of formula (X) thus obtained is then reacted with 1,4-diaminobenzene-2-sulfonic acid giving compound (XI) (the second half of this suspension being kept for subsequent reaction with compound (XI)). The 1,4-diaminobenzene-2-sulfonic acid is conveniently suspended in water at a temperature about 35 - 45°C, preferably about 38 - 42°C, whereupon caustic alkali is added, preferably an aqueous lithium hydroxide solution, until the pH reaches a value of about 8 and solution takes place. This solution is then added to the aforesaid suspension of the compound of formula (X) at a temperature of about 40 - 55°C, preferably about 45 - 50°C, and after this addition the pH, which has reached a value of about 5.5 - 6, is kept at this value by addition of caustic alkali, preferably aqueous lithium hydroxide solution. Normally, the reaction is finished after about 4 - 5 hours.

The second half of the aforesaid suspension of the compound of formula (X) is then given to the suspension obtained in the foregoing operation, conveniently at a temperature of about 40°C, whereby the pH raises to about 6 - 7. The mixture is then heated, conveniently to a temperature of about 65 - 80°C, preferably about 72 - 75°C, which results in a decrease of the pH to about 5.5 - 6 at which value the pH is kept by addition of caustic alkali, preferably aqueous lithium hydroxide solution. In normal circumstances the reaction is finished after about 5 - 6 hours.

The suspension obtained in the foregoing operation is treated with 1-methylamino-ethyl-2-sulfonic acid which is conveniently utilized in the form of an aqueous solution of its sodium salt, or any other amine. This solution is added to the aforesaid suspension at a temperature of about 80 - 90°C, preferably about 82 - 86°C, whereby the pH rises from about 5 to about 10 and is then brought to and kept at about 8.2 - 8.6, preferably about 8.4, by addition of caustic alkali, preferably aqueous lithium hydroxide solution. The reaction is completed after 4 - 5 hours.

The resulting solution contains a mixture of compounds of formulae (I), (II) and (III), as defined hereinabove, in the form of alkali metal salts, preferably salts with lithium and sodium. This solution can be purified by conventional procedures, e.g. by filtration and/or ultrafiltration, and concentrated. If desired, the salts can be converted into the corresponding compounds of formulae (I), (II) and (III) or into other salts according to methods which are readily available to those skilled in the art. Conversely, compounds of formulae (I), (II) and (III) which may have been obtained by carrying out the synthesis described hereinabove in a slightly different manner, can be converted into salts. Suitable salts include the aforementioned lithium and sodium salts as well as potassium salts, ammonium salts etc. and inner salts.

The compounds according to the invention or their salts may be used for dyeing cationic dyeable materials such as: homo- or mixed-polymers of acrylonitrile, acid modified polyester or polyamide; wool; leather including low affinity vegetable-tanned leather; cotton; bast fibers such as hemp, flax, sisal, jute, coir and straw; regenerated cellulose fibers, glass or glass products comprising glass fibers; and substrates comprising cellulose for example paper and cotton. They may also be used for printing fibers, filaments and textiles comprising any of the above mentioned materials in accordance with known methods. Printing may be effected by impregnation of the material to be printed with a suitable printing paste comprising one or more compounds of the present invention. The type of printing paste employed, may vary depending on the material to be printed. Choice of a suitable commercially available printing paste or production of a suitable paste, is routine for one skilled in the art. Alternatively the compounds of the present invention may be used in the preparation of inks suitable for example for jet printing, in accordance with conventional methods.

Most preferably, the dyestuffs are used for dyeing or printing of paper e.g., sized or unsized, wood-free or wood-containing paper or paper-based products such as cardboard. They may be used in continuous dyeing in the stock, dyeing in the size press, in a conventional dipping or surface coloring process. The dyeing and printing of paper is effected by known methods.

The dyeings and prints and particularly those obtained on paper, show good fastness properties.

The compounds of formula (I) may be converted into dyeing preparations. Processing into stable liquid, preferably aqueous, or solid (granulated or powder form) dyeing preparations may take place in a generally known manner. Advantageously suitable liquid dyeing preparations may be made by dissolving the dyestuff or its salt in suitable solvents such as formamide, dimethylformamide, urea, glycols and ethers thereof, dextrin or addition products of boric acid with sorbitol which may be used together with water, optionally adding an assistant, e.g. a stabilizer. Such preparations may be obtained, for example, as described in French patent specification No. 1,572,030.

The compounds of formula (I) (in the corresponding salt form) have good solubility especially in cold water. Owing to their high substantivity the compounds of the present invention exhaust practically quantitatively and show a good build-up power. They can be added to the stock directly, i.e. without previously dissolving, as either a dry powder or granulate, without reducing the brilliance or the yield of color. They can also be used in soft water without loss of yield. They do not mottle when applied on paper, are not inclined to give two-sided dyeing on paper and are practically insensitive to filler or pH variations. They operate over a broad pH range, in the range of from pH 3 to 10. When producing sized or unsized paper, the waste water is essentially colorless. This feature, which is extremely important from an environmental view-point, when compared with similar known dyes, shows a marked improvement. A sized paper dyeing when compared with the corresponding unsized paper dyeing does not show any decrease in strength.

The paper dyeings or printings made with the compounds, in particular the metal-free forms, according to the invention are clear and brilliant and have very good light fastness: On exposure to light for a long time, the shade of the dyeing fades tone in tone. They show very good wet fastness properties; being fast to water, milk, fruit juice, sweetened mineral water, tonic water, soap and sodium chloride solution, urine etc. Furthermore, they have good alcohol fastness properties. The wet fastness properties are improved compared to known dyes showing otherwise similar properties. They do not exhibit a tendency towards two-sidedness.

Paper dyed or printed with the compounds of the present invention can be bleached oxidatively, a feature which is important for the recycling of waste and old paper/paper products. This property, together with the improved backwater results and wet-fastness, shows a marked improvement over known dyes having otherwise similar properties.

The compounds of the present invention may also be used to dye paper containing wood-pulp where even dyeings, having good fastness properties are obtained. Furthermore, they may be used for the production of coated paper in accordance with known methods. Preferably when coating, a suitable filler, for example kaolin, is employed in order to give a one-side coated paper.

The compounds of the present invention are also suitable for dyeing in combination with other dyes for example other cationic or anionic dyes. The compatibility of the compounds of the present invention when used as a dye in mixtures with other commercially available dyes, may be determined according to conventional methods. The thus obtained dyeings have good fastness properties.
The compounds of the present invention are also suitable for the preparation of ink jet inks.

The invention further provides a substrate which has been dyed or printed with a compound of the present invention. The substrate may be selected from any of the above mentioned substrates. A preferred substrate is a substrate comprising cellulose such as cotton or paper or a paper based product. The most preferred substrate is paper or a paper based product.

The following Examples further serve to illustrate the present invention. In these Examples all parts and all the percentages are by weight or volume, and the temperatures given are in degrees Celsius, unless indicated to the contrary.

### EXAMPLE 1

### a) Preparation of intermediate A

13 parts of the compound with the formula 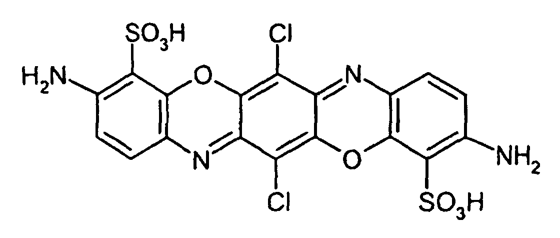 are dissolved in 220 parts water and brought to pH 8 - 8.5 with lithium hydroxide. 50 parts of ice are added, the solution is divided in two equal parts and kept at 4°C. 2.4 parts of 2,4,6-trichloro-s-triazine are dispersed in 10 parts water and 5 parts ice as well as a dispersing agent are added. The suspension is stirred for 30 minutes and added portion wise to the first part of the solution of the dioxazine compound whereby the pH value is kept at 8 - 8.5 with lithium hydroxide and the temperature at 4 - 7°C. Then the mixture is stirred for 2 hours and the pH is lowered to 5.5 - 6 with acid. After addition of the second part of the dioxazine solution, the mixture is heated to 70 - 75°C. The reaction is finished after 6 hours. 14.3 parts of a compound with the formula 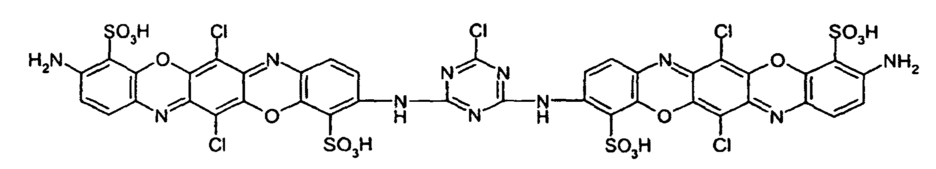 (intermediate A) in 312 parts of water is obtained in the form of the lithium salt.

### b) Reduction of intermediate A

14.3 parts of the intermediate A in 312 parts of water are put into a reaction vessel and 128.4 parts hydrochloric acid are added under nitrogen atmosphere while stirring vigorously. The mixture is stirred for a further 30 minute and 1.34 parts iron powder are added within 3 - 4 hours. The reaction continues over night and the brown suspension is filtered. 14.4 parts of the reduced intermediate A (corresponding to formula (II) in which X is Cl and R₄ and R₆ are H) are obtained.

### c) Preparation of intermediate B

7.1 parts 1-amino-benzene-2-sulfonic acid are dissolved in 110 parts water and neutralized (pH 7 - 7.5) with lithium hydroxide. 8 parts of 2,4,6-trichlorotriazine are dispersed in 40 parts ice water and dispersing agent by stirring during 30 minutes. The suspension is added to the solution of the aminobenzenesulphonic acid salt portionwise within 1 hour at 4 - 7°C and at pH 4.8 - 5.2. The mixture is stirred over night at 0 - 5°C. 13.2 parts of the compound with the formula 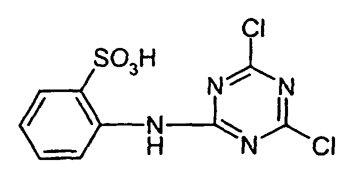 (intermediate B) in 227 parts of water are obtained in the form of the lithium salt.

### d) Condensation of reduced intermediate A with intermediate B

14.4 parts of the reduced intermediate A are stirred under nitrogen atmosphere into 240 parts water. The reaction mixture from step c) is added at once and the pH adjusted to a value 4 within 10 - 15 minutes. The mixture is then heated to 60°C and stirred for 16 hours at pH 4 and 60°C. The temperature is raised to 70°C and the reaction continued for about 1.5 hours. 21.1 parts of the compound with the formula 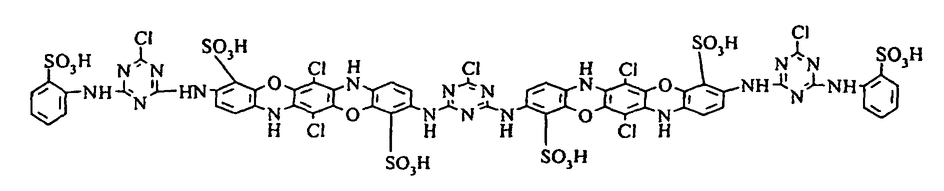 (intermediate C) in 619 parts of water as a colloidal solution are obtained.

### e) Condensation of intermediate C with 1-methyl-amino-ethyl-2-sulfonic acid

640 parts of the colloidal solution obtained in step d) are put into a reaction vessel and 10 parts 1-methylamino-ethyl-2-sulfonic acid in the form of the sodium salt as a solution is added. The mixture is heated to 80°C and left during 1 hour and over night at 80°C and pH 8 (if necessary, by addition of lithium hydroxide) with normal air present. At the same time as the condensation takes place, the leuco form is oxidized and the dyestuff is precipitated with sodium chloride, filtered and dried at 60°C in vacuum. 24.8 parts of the blue dyestuff with the formula 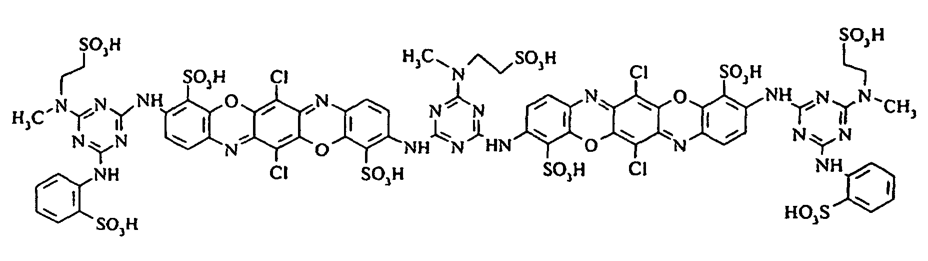 is obtained in the mixed lithium/sodium salt form. λₘₐₓ is 564.4 nm (in water and 1 % acetate).

### EXAMPLE 2 (not according to the invention)

Following the procedure of Example 1 the condensation of intermediate C was performed with diethanolamine.
640 parts of the colloidal solution of step d) are put into a reaction vessel and 6 parts of diethanolamine are added. The mixture is heated to 80 - 85°C and left over night at 80 - 85°C and pH 8 (if necessary by addition of lithiumhydroxide) with normal air present. At the same time as the condensation takes place, the leuco form is oxidized. The reaction mixture is cooled to 50°C and filtered. 650 parts process solution are ultrafiltrated at 40 - 50°C using a suitable membrane (e.g. G10; G20; G50) and concentrated up to 400 parts solution.
The obtained liquid dyestuff formulation contains only traces of the remaining inorganic salts and exhibits perfect storage stability. λₘₐₓ is 555.7 nm (in water and 1 % acetate).

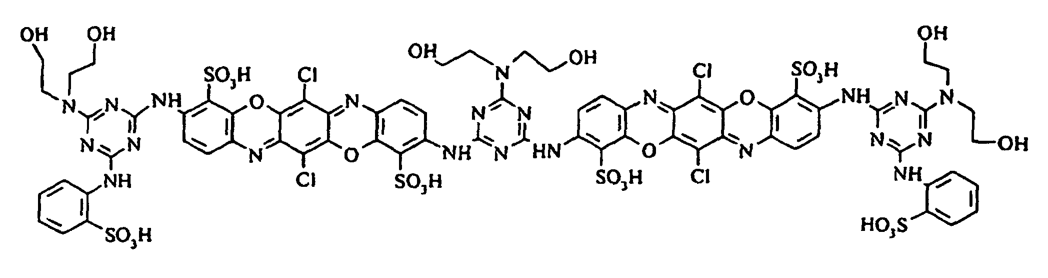

### EXAMPLES 3 - 18

Following the procedure of Example 1 or an analogous procedure with similar reactants, further dyestuffs according to formula (Ic) 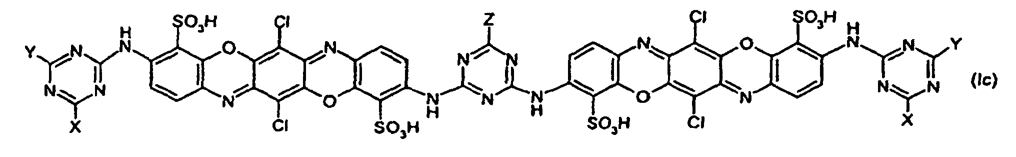 can be obtained which are illustrated in the following Table 1.

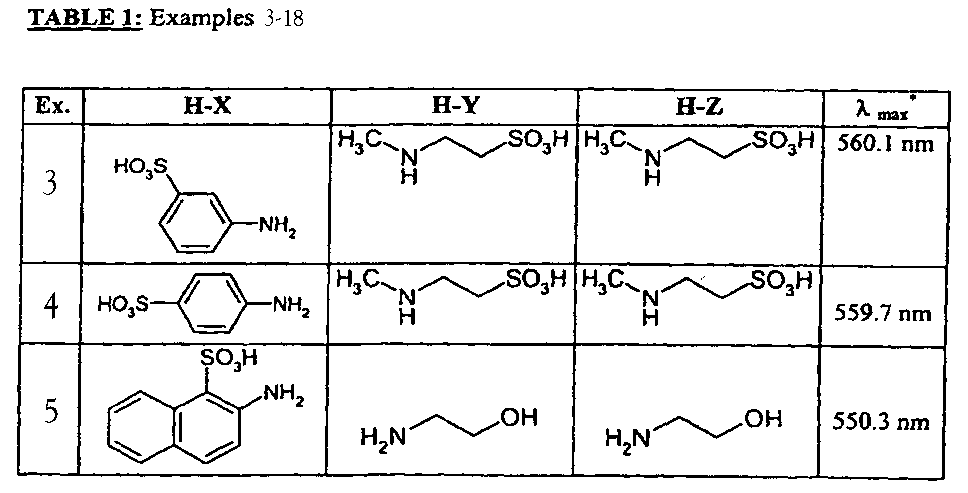

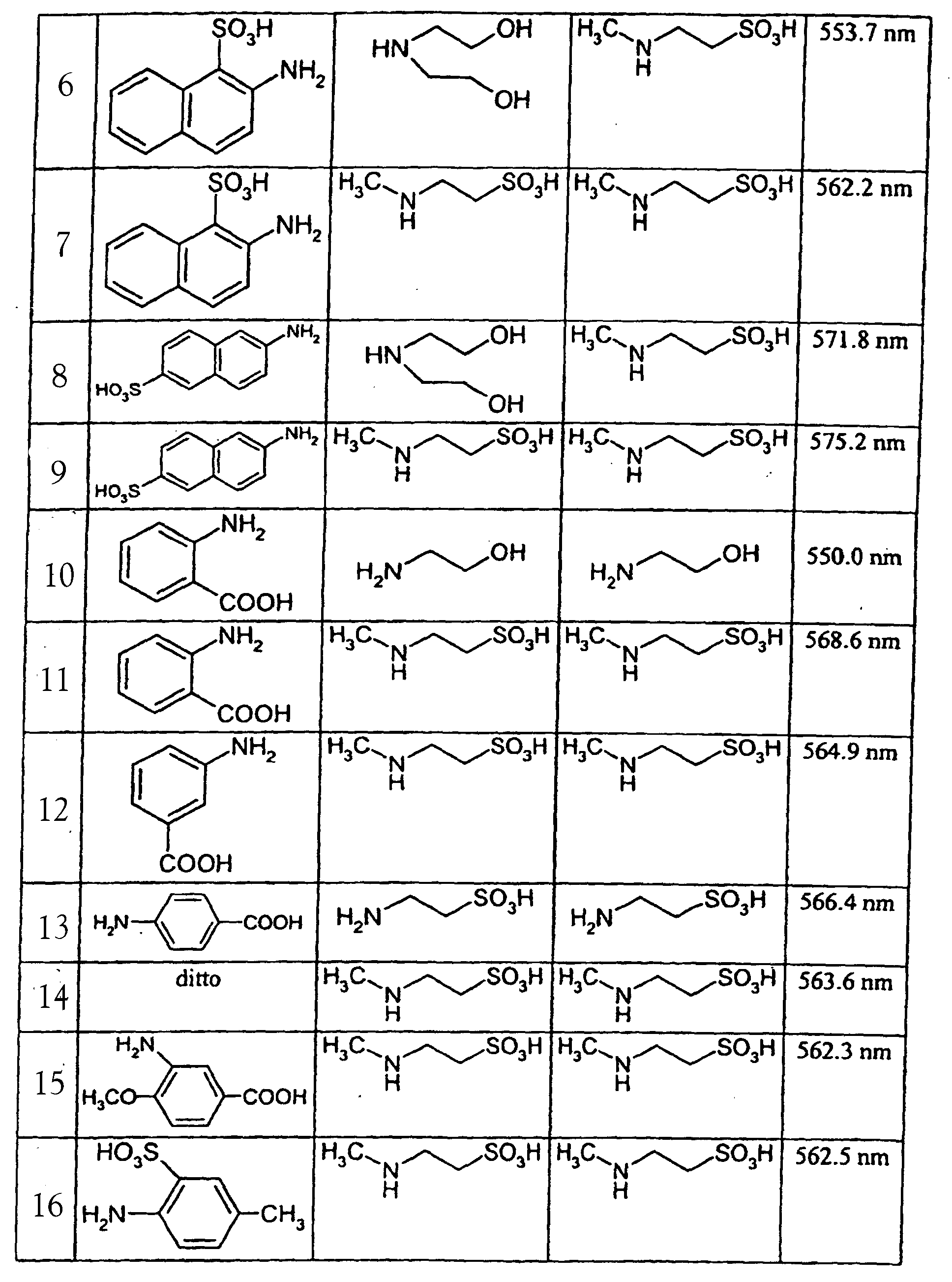

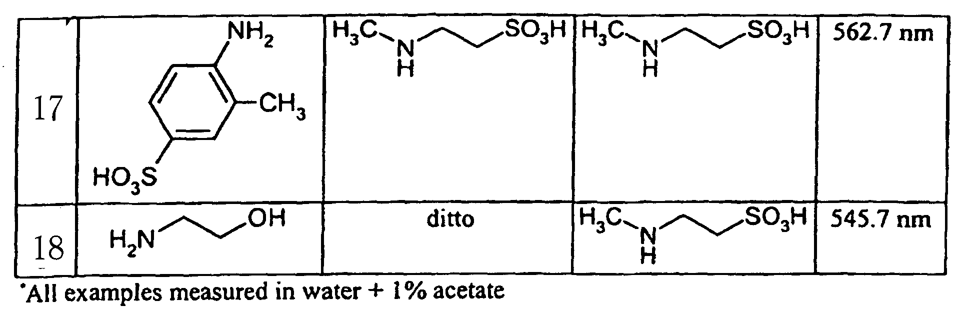

All these dyestuffs dye cotton or paper in brilliant reddish blue or blue shades with excellent fastnesses.

### EXAMPLE 19

a) 82.0 parts of 3,10-diamino-6,13-dichloro-4,11-triphendioxazine disulphonic acid are added, with stirring and at 20 - 25°C, to 750 parts of water. At the same temperature a solution of 16.5 parts of lithium hydroxide monohydrate, in the form of 100 parts of a 4 N solution, is added over 2 - 3 hours at pH 8 - 8.5. 1100 parts of a solution are obtained.
b) 30.4 parts of 2,4,6-trichlorotriazine are added in portions, with stirring and at 1 - 4°C, to a mixture of 67 parts of water, 0.3 parts of a conventional surfactant and 22 parts of ice. Stirring is continued for 5 minutes, the temperature is kept at 1 - 4°C by addition of further 37 parts of ice, and stirring is continued for further 15 minutes. 150 parts of a suspension are obtained.
c) The solution obtained in a) is brought to a temperature of 5 - 8°C by addition of 240 parts of ice. The suspension obtained in b) is added with stirring over 2 hours, the pH is maintained at 8.0 - 8.3 by continuous addition of 4 N lithium hydroxide solution, and the temperature is kept at 5 - 8°C by adding 75 parts of ice portion-wise. Stirring is continued for one hour at the same pH range, and the temperature rises to 10 - 12°C. The volume of the resulting suspension is about 1700 parts.
d) 15.5 parts of 1,4-diaminobenzene-2-sulfonic acid are suspended in 200 parts of water. The pH is about 3. The suspension is heated to 40°C, and the pH is brought to 8 by addition of 4 N lithium hydroxide solution. 230 parts of a dark-brown solution are obtained.
e) One half (about 850 parts) of the suspension obtained in c) is heated to about 45°C, the pH decreases to 6 - 7. The solution obtained in d) is added, whereby the pH starts to decrease further. When the pH reaches 5.5 - 6, it is kept at that value by addition of 4 N lithium hydroxide solution. The mixture is heated to 50° and kept at this temperature until the reaction is complete (about 4 hours). 1150 parts of a suspension are obtained.
f) The second half of the suspension obtained in c) is heated to 40°C, the pH is decreased to 6 - 7, and added to the suspension obtained in e). The vessel in which the suspension obtained in c) had been prepared is rinsed with 200 parts of water, and the rinsings are added to the mixture. The pH rises to 6 - 6.5, the mixture is heated to 70 - 72°C, the pH decreases again to 5.5 - 6 and is kept at that value by addition of 4 N lithium hydroxide solution. After 5 - 6 hours at 72 - 75°C the reaction is complete. 2200 parts of a suspension are obtained.
g) 75 parts of 1-methylamino-ethyl-2-sulfonic acid, in the form of a 39 % aqueous solution of its sodium salt are added to the suspension obtained in f). The pH rises from 5 to 10. The temperature is kept at 84°C (+/- 2°C), and the pH is kept at 8.4 (+/- 0.2) by addition of 4 N lithium hydroxide solution. After about 4 - 5 hours the reaction is complete. 2350 parts of a solution are obtained.
h) 16 parts of a conventional filtration aid are added to the solution obtained in g). After stirring for about 20 minutes the suspension is filtered by suction. The filtrate is subjected, at 48 - 50°C, to ultrafiltration using an appropriate conventional membrane and 9500 parts of demineralised water and thereafter concentrated to a volume of 2000 parts. The resulting stable dark-blue solution contains about 6 % of dyestuffs, namely a mixture of about
   a) 30 - 40 % of the following compound 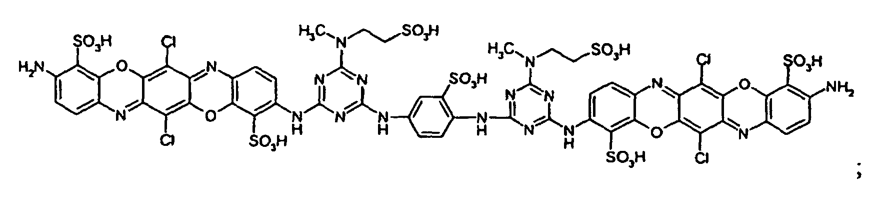
   b) 30 - 47 % of the following compound 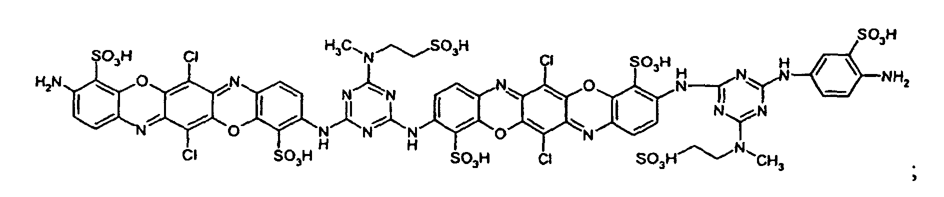
   c) 1.5 - 8 % of the following compound 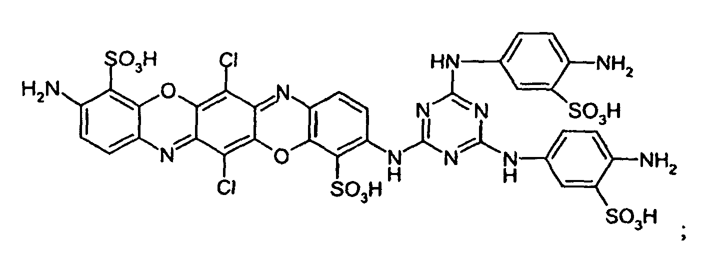
   d) 2 - 11 % of the following compound 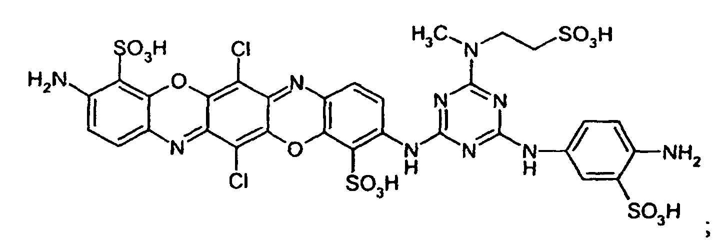 and
   e) 12.5 - 23.5 % of the following compound 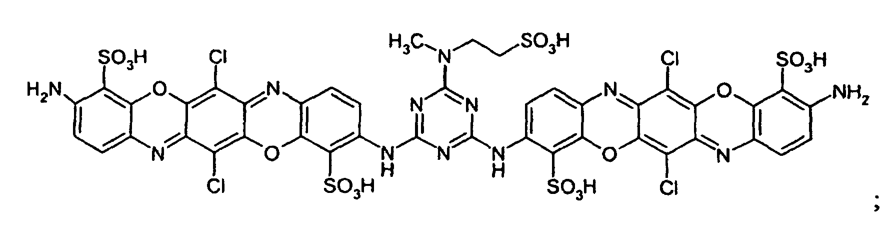
in the form of their lithium/sodium salts, corresponding to about 5.5 % of the free acids. The λₘₐₓ value is 572.1 nm (in water and 1% acetate).

### Example 20 (not according to the invention)

The steps a) - f) are analogous to Example 19.
In step g) instead of using 75 parts of 1-methylamino-ethyl-2-sulfonic acid solution 22.5 parts of 1-amino-ethyl-2-sulfonic acid in 50 parts water are used under the same condition as mentioned in step g).
After 4 - 5 hours the reaction is complete. The dyestuff is precipitated with sodium chloride, filtered and dried at 60°C in vacuum. 130 parts of the dyestuff are obtained. λₘₐₓ is 571.5 nm (in water and 1% acetate).

### TABLE 2 Examples 21 - 33

In analogy to the Example 19 further dyestuffs can be obtained. But instead of using 1-methylamino-ethyl-2-sulfonic acid as in Example 19 step g) the following substances H-Z as illustrated in Table 2 are used. Further analogously or instead as in step d) the following compounds S are used in the corresponding molar amount.

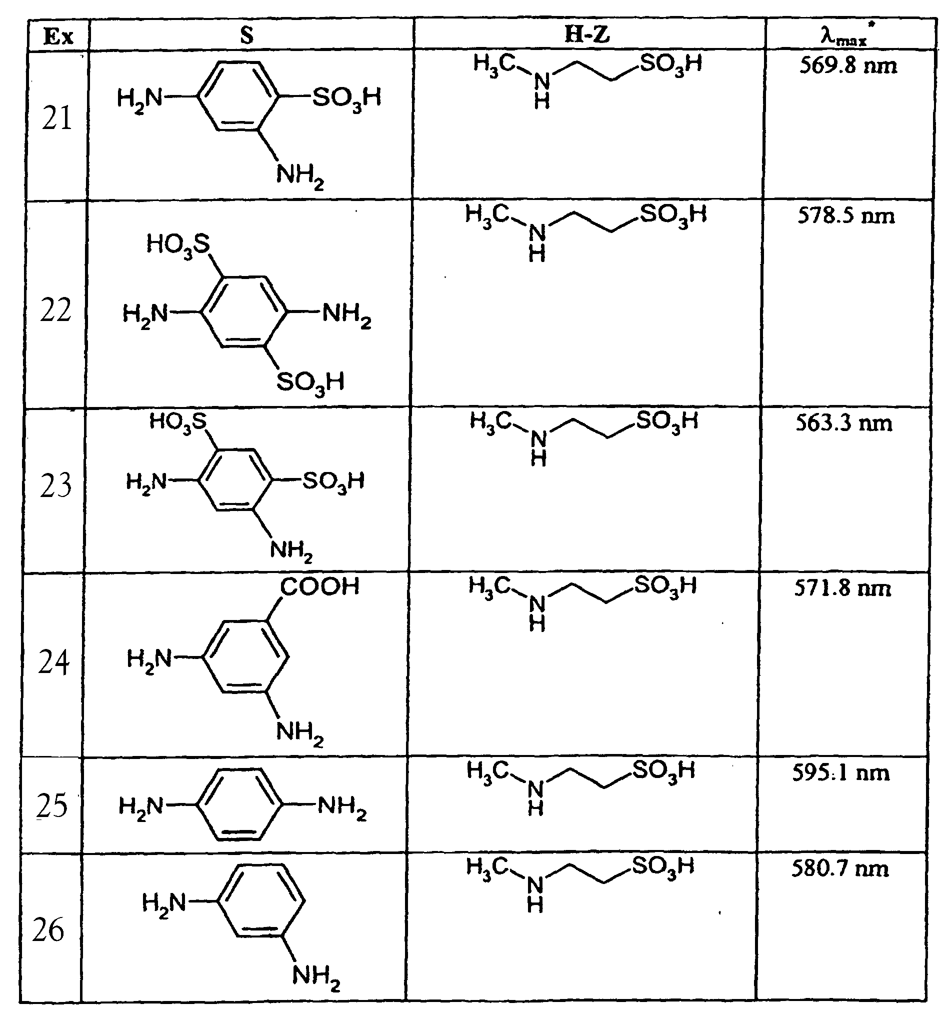

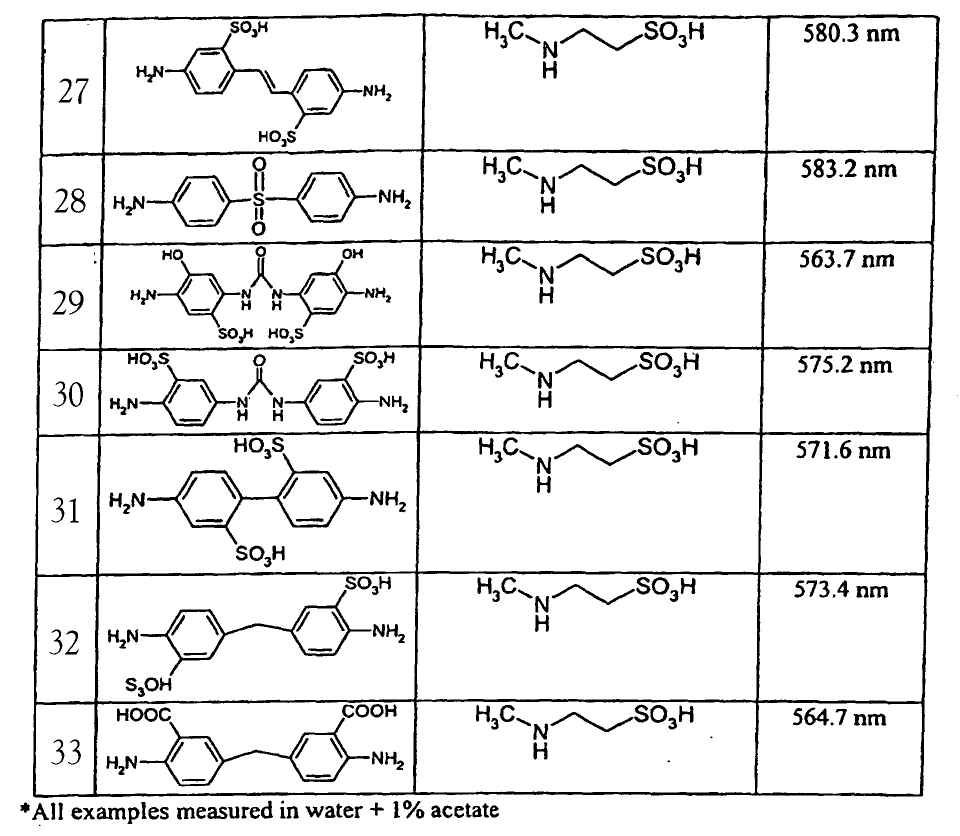

The application of the dyestuffs is illustrated in the following Application Examples.

### APPLICATION EXAMPLE A

70 parts of chemically bleached sulfite cellulose obtained from pinewood and 30 parts of chemically bleached cellulose obtained from birchwood are beaten in 2000 parts of water in a Hollander. 0.2 parts of the dyestuff solution obtained in step h) of Example 19 are sprinkled into this pulp. After mixing for 10 minutes, paper is produced from this pulp. The absorbent paper obtained in this way is dyed a brilliant blue. The waste water is colorless.

### APPLICATION EXAMPLE B

0.2 parts of the dyestuff solution obtained in step h) of Example 19 are dissolved in 100 parts of water. This solution is added to 100 parts of chemically bleached sulfite cellulose which have been ground with 2000 parts of water in a Hollander. After mixing thoroughly for 15 minutes, resin size and aluminium sulfate are added thereto. Paper produced in this way has a brilliant blue nuance and exhibits perfect light and wet fastness.

### APPLICATION EXAMPLE C

An absorbent length of unsized paper is drawn at 40 - 50°C through a dyestuff solution having the following composition:

| | |
|---|---|
| 0.3 | parts of the dyestuff solution obtained in step h) of Example 19; |
| 0.5 | parts of starch; and |
| 99.0 | parts of water. |

The excess dyestuff solution is squeezed out trough two rollers. The dried length of paper is dyed a brilliant blue shade. The paper dyeing obtained shows good fastness properties.

The dyestuff of Examples 1 - 18 and 20 - 33 may also be used for dyeing by a method analogous to that of Application Examples A to C. The paper dyeings obtained show good fastness properties.

### APPLICATION EXAMPLE D

2 parts of the dyestuff solution obtained in step h) of Example 19 are dissolved in 1000 parts of demineralised water at 40°C. 100 parts of a pre-wetted cotton textile substrate are added, and the bath is heated to the boiling point over 30 minutes and held at the boil for one hour.

Any water which evaporates during dyeing is replaced continuously. The dyed substrate is removed from the bath and, after rinsing and drying, a brilliant blue dyeing is obtained which has good light- and wet-fastness properties. The dyestuff exhausts practically totally onto the fiber, and the waste water is almost colorless.

In a similar manner as described in Application Example D the dyestuffs according to Examples 1 - 18 and 20 - 33 may be used for dyeing cotton.

### APPLICATION EXAMPLE E

100 parts freshly tanned and neutralized chrome leather are agitated for 30 minutes in a vessel with a liquor consisting of 250 parts of water at 55°C and 0.5 parts of the dyestuff of Example 1 and then treated in the same bath for 30 minutes with 2 parts of an anionic fatty licker based on sulphonated train oil. The leather is then dried and prepared in the normal way, giving a leather evenly dyed in a brilliant blue shade.

By a method analogous to that described in Application Example E the dyestuffs according to Examples 2 - 33 may be used for dyeing leather.

Further vegetable-tanned leathers of low affinity may be dyed using the dyestuffs as described herein in accordance with known methods.

### APPLICATION EXAMPLE F

Water is added to a dry pulp in a Hollander, said dry pulp consisting of 60 % (by weight) of mechanical wood pulp and 40 % (by weight) of unbleached sulfite cellulose, and the slurry is beaten in order to obtain a dry content slightly exceeding 2.5 % and having a beating degree of 40° SR (degrees Schopper-Riegler). The slurry is then exactly adjusted to a high density dry content of 2.5 % by adding water. 5 parts of a 2.5 % aqueous solution of the dyestuff obtained in step h) of Example 19 are added to 200 parts of the above resulting slurry. The mixture is stirred for about 5 minutes and, after addition of 2 % (by weight) resin size and then 4 % (by weight) alum (based on the dry weight), stirring is continued for a few minutes until the mixture is homogeneous. The resulting pulp is diluted with 500 parts of water to a volume of 700 parts and then used for the production of paper sheets by suction on a sheet former. The resulting paper sheets are dyed a brilliant blue. The waste water exhibits a substantially low residual dye concentration.

In a similar manner as described in Application Example F the dyestuffs according to Examples 1 - 18 and 20 - 33 may be used for dyeing paper.

### APPLICATION EXAMPLE G

100 parts of cotton tricot, which have been dyed with the dyestuff of Example 1 analogously to the method of example F in ca. 1/1 standard depth, are mixed without intermediate drying in 1000 parts of tap water at 25°C with 5 parts of sodium chloride and 4 parts of an after-treatment agent obtained from the reaction of diethylenetriamine with dicyandiamide. The pH value of the dye bath is set at 6.5 - 7. The bath is heated to 60°C over the course of 20 minutes, and this temperature is maintained for a further 20 minutes. Afterwards, the material is'rinsed with cold tap water. The red cotton dyeing which has been after-treated in this way has perfect washing fastness and very good light fastness.

In a similar manner as described in Application Example G the dyestuffs according to Examples 2 - 33 may be used for dyeing cotton.

### APPLICATION EXAMPLE H

A cotton dyeing produced with the dyestuff of Example 1 analogously to the method of example F in 1/1 standard depth, is impregnated on a padder with a solution, which contains 100 g/l of an after-treatment agent obtained by reacting the after-treatment agent of Application Example G with dimethyloldihydroxyethyleneurea and a hardening catalyst, and it is squeezed out to a pick-up of ca. 80 %. It is subsequently shock-dried for 45 seconds on a stenter at a temperature of 175°C - 180°C. The yellow cotton dyeing thus obtained is notable for its perfect washing fastness. At the same time, there is a considerable improvement in the creasing fastness, and reduced swelling value of the cellulosic fibres.

In a similar manner as described in Application Example H the dyestuffs according to Examples 2 - 33 may be used for dyeing cotton.

### APPLICATION EXAMPLE I

Water is added to a dry pulp in a Hollander consisting of 50 % (by weight) of chemically bleached sulphite cellulose obtained from pinewood and 50 % (by weight) of chemically bleached sulphite cellulose obtained from birchwood, and the slurry is ground until a degree of grinding of 35° SR is reached. The slurry is then adjusted to a high density dry content of 2.5 % by adding water, and the pH of this suspension is adjusted to 7.

10 parts of a 0.5 % aqueous solution of the dyestuff obtained in step (h) of Example 19 are added to 200 parts of the above resulting slurry, and the mixture is stirred for 5 minutes. The resulting pulp is diluted with 500 parts water and then used for the production of sheets by suction on a sheet former. The paper sheets thus obtained have a brilliant blue shade.

By a method analogous to that described in Application Example I further dyestuffs may be used consisting of any one of the dyestuffs of Examples 1 - 18 and 20 - 33. In all cases, paper sheets are formed having a brilliant blue shade.

### APPLICATION EXAMPLE J

2.5 parts of the dyestuff obtained in Example 19 are dissolved with stirring at 25°C in a mixture of 20 parts diethyleneglycol and 77.5 parts water to obtain a printing ink suitable for ink jet printing.

This application method is suitable for all Examples.

## Claims

1. Compounds of formula (I) 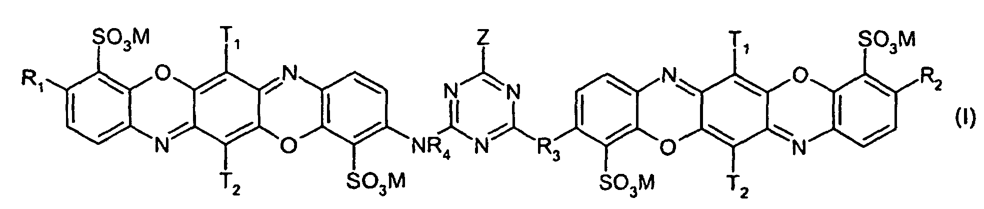 wherein
R₁ signifies -NH₂ or 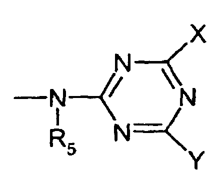 wherein
R₅ signifies hydrogen or C₁₋₄-alkyl,
X and Y independently signify halogen or hydroxy or C₁₋₃-alkoxy or phenoxy or the radical of a cyclic, an aliphatic, an araliphatic or an aromatic amine linked over the amine-nitrogen and optionally substituted by hydroxy, carboxy, alkoxy, alkyl and/or sulphonic acid groups or the rest of a heterocyclic amine linked over the amine-nitrogen,
Z signifies a radical of the formula 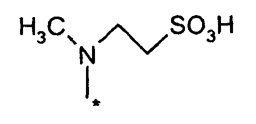 wherein the aterix show the point of attachement,
R₂ signifies -NH₂ or 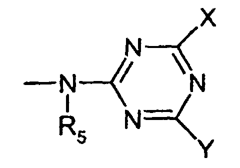 with the same definitions for R₅, X and Y as above or
R₂ is the rest (b) 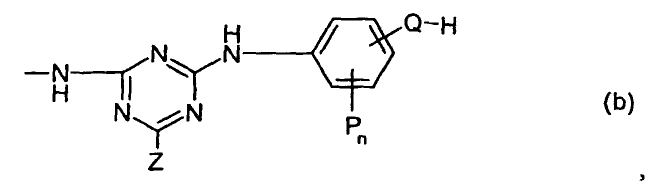 wherein
P signifies -SO₃H, -COOH or -OH,
Q signifies a -NH- radical or a group selected from 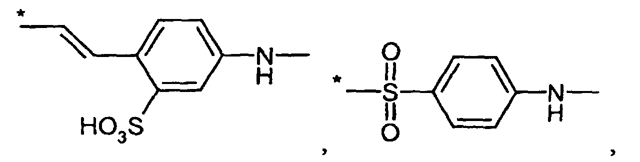 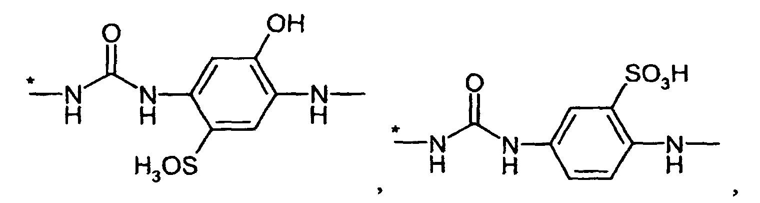 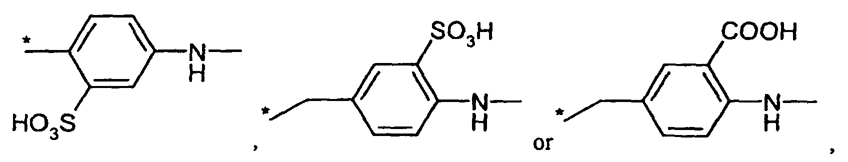 wherein the asterisk marks the bond attached to the phenyl ring,
Z signifies the same as above,
n has the value 0, 1, 2,
R₃ signifies -NR₆
in which R₆ signifies hydrogen or a C₁₋₄-alkyl radical
or
R₃ is the rest (a) 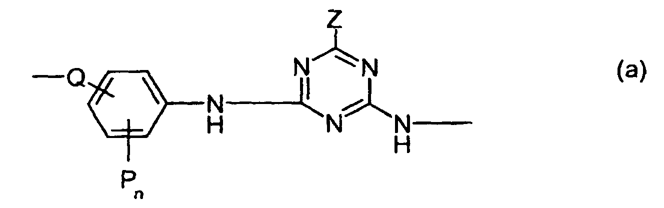
wherein all substituents have the same meanings as defined above,
R₄ signifies hydrogen or C₁₋₄ -alkyl,
T₁ and T₂ independently from each other signify hydrogen, halogen, C₁₋₆-alkyl or C₁₋₄-alkoxy,
M signifies hydrogen or a cation;
with the provisos that
(i) when R₁ and R₂ is -NH₂ then R₃ is 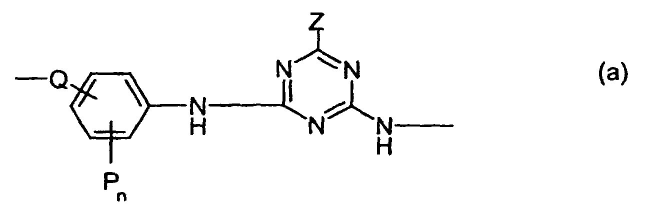 wherein P, Q, Z and n have the same meanings as defined above,
(ii) when R₁ is -NH₂ and R₂ is 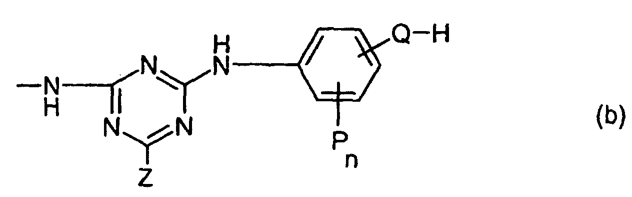
wherein P, Q, Z and n have the same meaning as defined above,
then R₃ is -NH-,
salts thereof and mixtures of such compounds and/or salts.

2. A compound according to Claim 1,
wherein both R₁ and R₂ signify 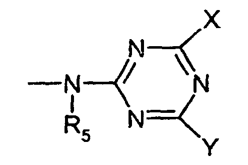 wherein
R₅ signifies hydrogen or C₁₋₄-alkyl,
and
R₃ signifies -NR₆
in which R₆ signifies hydrogen or a C₁₋₄-alkyl radical
salts thereof and mixtures of such compounds and/or salts.

3. A compound or mixture according to Claims 1 to 2, wherein the radicals X, Y and Z contain no chromophoric group.

4. A compound or mixture according to anyone of Claims 1 to 3, wherein any amine H-X, H-Y or H-Z has a molecular weight in the range of 50 to 500, preferably 50 to 400.

5. A compound or mixture according to anyone of Claims 1 to 4, wherein any amine H-X, H-Y or H-Z contains no ring or 1 to 4 rings, preferably no ring or 1 to 2 rings.

6. A compound or mixture according to anyone of Claims 1 to 5, wherein at least one amine radical X, Y and Z contains at least one hydrophilic group.

7. A compound or mixture according to Claim 6, wherein each hydrophilic group or substituent is independently anionic, cationic or non-ionogenic.

8. A compound or mixture according to anyone of Claims 1 to 7 **characterized in that** H-X and H-Y are independently ammonia and aliphatic amines, preferably substituted with a hydroxy, carboxy or sulphonic acid group, e.g. ethanolamine, diethanolamine, isopropanolamine, diisopropanolamine, 2-amino-hydroxypropane, glycine, N-methylethanolamine, 3-methoxy-propylamine, 1-aminoethyl-2-sulfonic acid and most preferably, 1-methylamino-ethyl-2-sulfonic acid; aromatic amines, preferably substituted with a carboxy, alkoxy and/or sulphonic acid group, e.g. aniline, 4- or 3-sulpho-aniline, 4- or 3-carboxy-aniline; and heterocyclic amines, e.g. morpholine, piperazine or hydroxyethylpiperazine; or N,N-diethylaminopropylamine and 1,2-diaminopropane and H-Z has the same meanings except the aromatic amines.

9. A compound or mixture according to anyone of Claims I to 8, wherein M is hydrogen or a colorless cation or M is a cationic portion in a substituent X, Y or Z containing a cationic charge, to form an inner salt.

10. Mixtures containing
a) a compound of formula (Ia) according to Claim 1 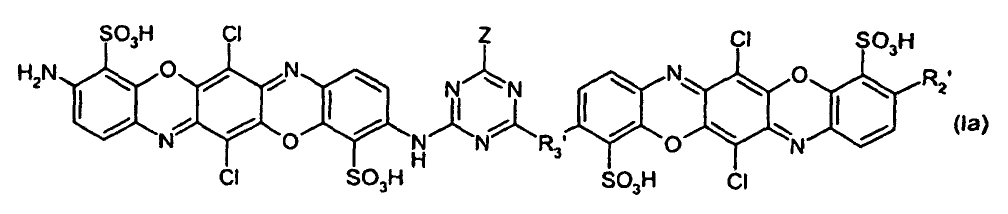 wherein R₃' is a divalent radical of formula (a), R₂' is -NH₂ and Z has the same meanings as defined in Claim 1.
b) a compound of the above formula (Ia) wherein R₃' is -NH-, R₂' is a radical of formula (b) and Z has the same meanings as defined in Claim 1.
c) a compound of formula (II) 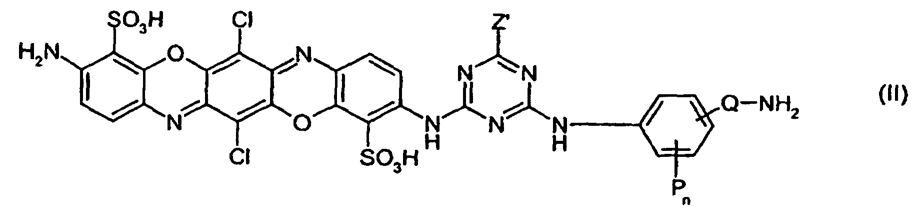 wherein Q, P and n have the same meaning as defined above and Z' has the same definition as Z, additionally Z' can be an aromatic amine, preferably for the component c) Z' is a radical of formula (c) 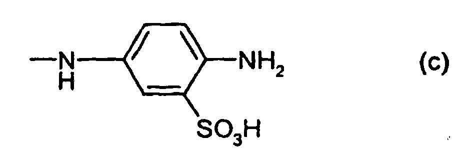
d) a compound of formula (II), wherein Z' has the same meanings as defined above, except halogen, preferably Z' is a radical of formula (d) 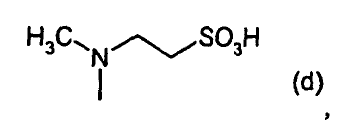
e) a compound of formula (III) 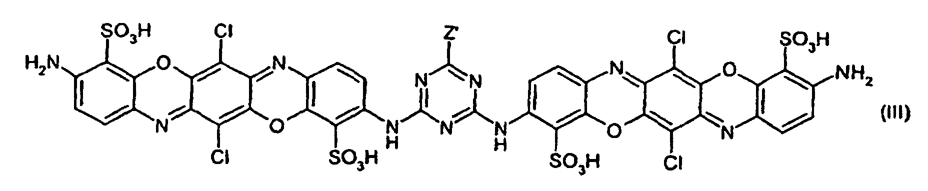 wherein Z' has the same definition as Z, additionally Z' can be an aromatic amine,
or salts of such compounds.

11. Mixtures according to Claim 10 containing approximately
30 - 40 % of component a),
30 - 47 % of component b),
1.5 - 8 % of component c),
2 - 11 % of component d), and
12.5 - 23.5 % of component e)
in the form of its alkali metal salts.

12. A process for the preparation of a compound of formula (Ib) 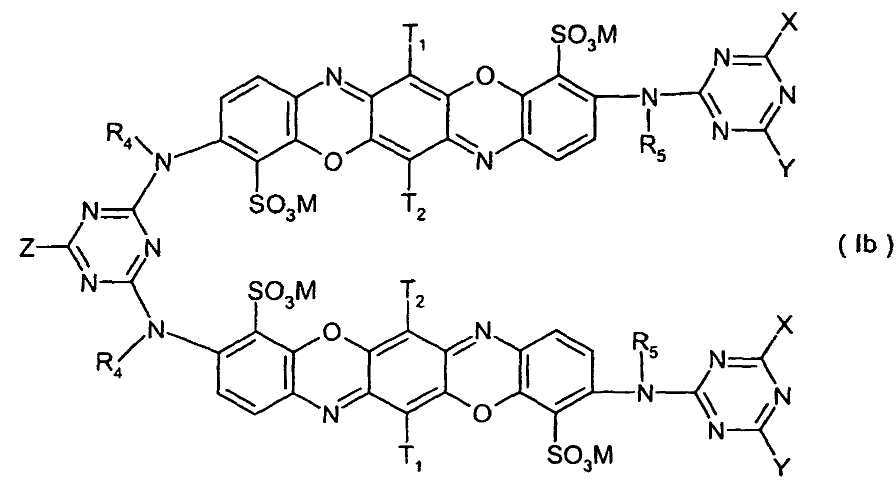 according to Claim 1 with the same definitions of the substituents as in Claim 1
wherein one mole of a compound of formula (IV) 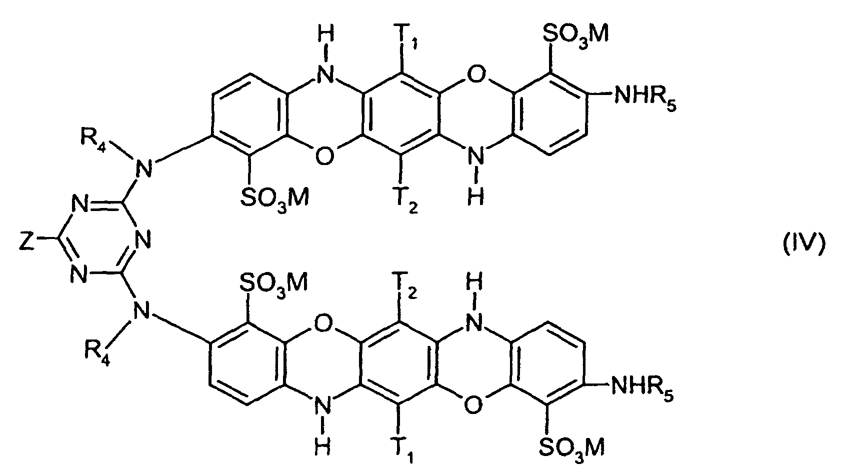 or a mixture of compounds of formula (IV) with the same definitions of the substituents as in formula (Ib) is reacted with two moles of at least one compound of formula (V) 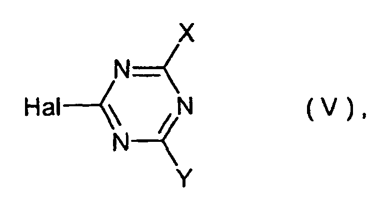 wherein Hal signifies halogen, preferably chlorine, and X and Y independently have the same meanings as defined in Claim 1, under dehydrohalogenating conditions, and, if in formula (Ib) anyone of X and Y has a significance other than halogen while in formula (V) the corresponding X or Y signifies halogen, and/or if in formula (Ib) Z has a significance other than halogen, the obtained condensation product of formula (VI) 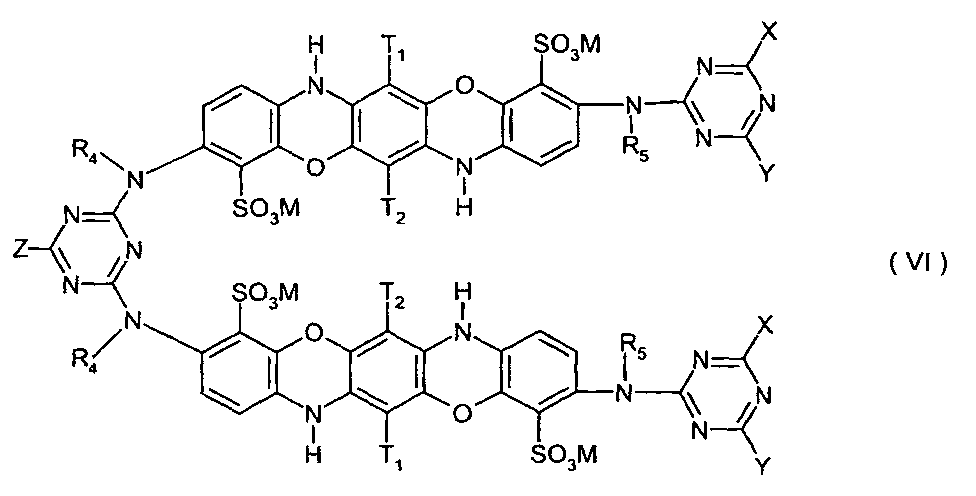 with the same definitions of the substituents as in formula (Ib) is reacted, before oxidation or simultaneously with oxidation, with at least one corresponding amine of formula H-X, H-Y and/or H-Z and/or an alkali metal C₁₋₃-alcoholate or phenolate, as required.

13. A process for the preparation of compounds of the formula (Ia), salts thereof and mixtures of such compounds and/or salts according to Claims 1 to 9, which comprises reacting a compound of formula (VIII) 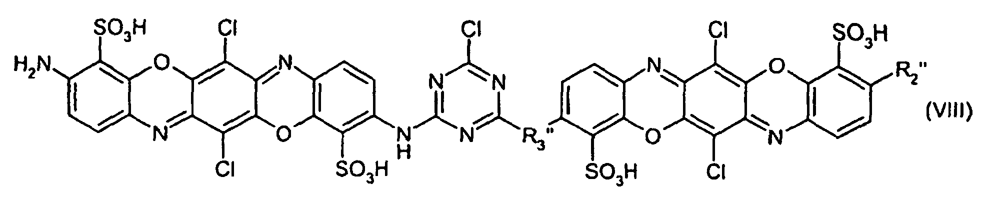 wherein either R₃" is a divalent radical of formula (a') 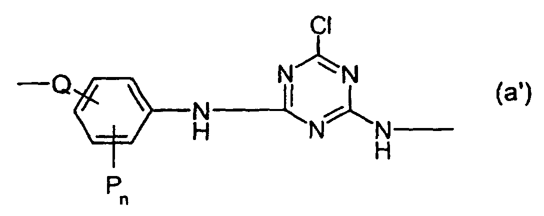 wherein P, Q and n have the same meanings as defined above
and R₂" is -NH₂; or
R₃" is -NH- and R₂" is a radical of formula (b') 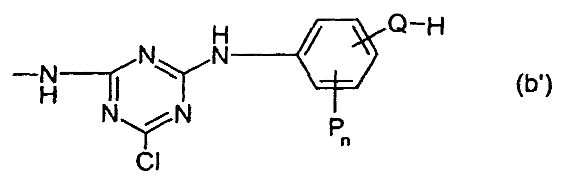 wherein P, Q and n have the same meanings as defined above
or a mixture of such compounds with an amine H-Z or H-Z' and, if desired, converting compounds of formula (I) thus obtained into salts or salts thus obtained into compounds of formula (I) or into other salts.

14. A process for the preparation of a mixture according to Claim 10 or Claim 11 which comprises
a) reacting a compound of formula (IX) 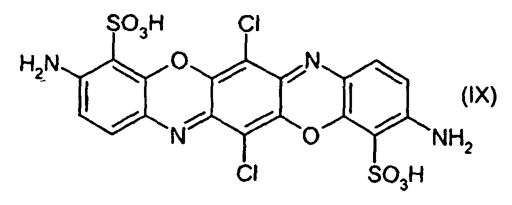 with about one equivalent of 2,4,6-trichlorotriazine;
b) reacting the compound of formula (X) 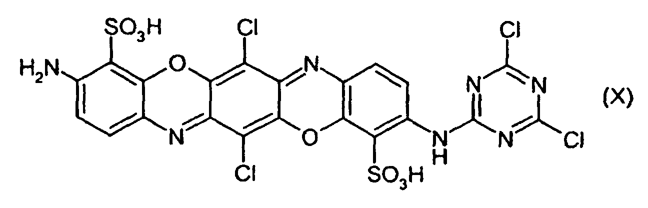 thus obtained with 1,4-diaminobenzene-2-sulfonic acid;
c) reacting the product thus obtained with another portion of the compound of the above formula (X);
d) reacting the product of the formula (VIII) thus obtained with an amine H-Z or H-Z'; and,
e) if desired, converting the mixture of compounds of formulae (I), (II) and (III) thus obtained into a mixture of salts or converting a mixture of salts thus obtained into a mixture of compounds of formulae (I), (II) and (III) or into a mixture of other salts.

15. Mixtures obtained by the process according to Claim 14.

16. A dye composition comprising at least one dye of formula (I) according to anyone of Claims 1 to 9 or salts thereof or a mixture of such compounds and/or salts, or a mixture according to Claims 10, 11, 14 or 15.

17. A liquid dyeing preparation containing a composition according to Claim 16 obtained by ultrafiltration.

18. Use of a compound of formula (I) according to anyone of Claim 1 to 9 or of a mixture according to Claims 10 or 11, optionally in the form of a composition according to Claim 16, as a dye for dyeing or printing substrates or as a component of printing inks.

19. Use according to Claim 18 wherein the substrate to be dyed or printed is paper or paper-based material.

20. A substrate dyed or printed according to Claim 18.

21. A substrate according to Claim 20 wherein the substrate which has been dyed or printed is paper or paper-based material.

22. Process for the preparation of ink-jet inks, **characterized in that** a compound of Claims 1 to 9 or salts thereof or a mixture of such compounds and/or salts, or a mixture according to Claims 10, 11, 14 or 15 is used

23. Ink-jet inks prepared by the process of Claim 12.

## Patentansprüche

1. Verbindungen der Formel (I) 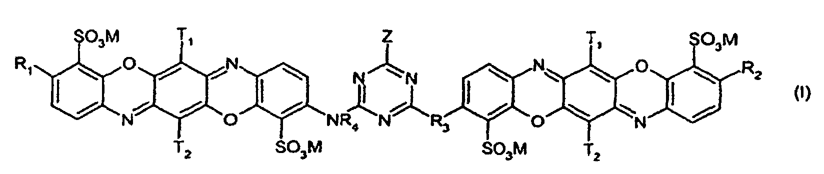 wobei die Variablen folgende Bedeutung haben:
R₁ bedeutet -NH₂ oder 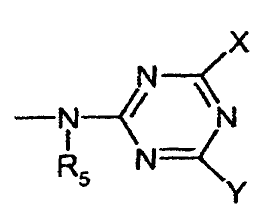 wobei
R₅ die Bedeutung Wasserstoff oder C₁₋₄-Alkyl hat und
X und Y unabhängig voneinander für Halogen oder Hydroxy oder C₁₋₃-Alkoxy oder Phenoxy oder den Rest eines cyclischen, aliphatischen, araliphatischen oder aromatischen Amins, der über Aminstickstoff gebunden und gegebenenfalls durch Hydroxy-, Carboxy-, Alkoxy-, Alkyl- und/oder Sulfonsäuregruppen substituiert ist, oder für den über Aminstickstoff gebundenen Rest eines heterocyclischen Amins stehen,
Z bedeutet einen Rest der Formel 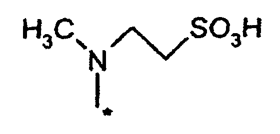 wobei der Stern die Verknüpfungsstelle zeigt,
R₂ bedeutet -NH₂ oder 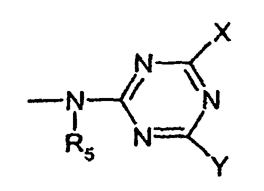 wobei R₅, X und Y die obengenannte Bedeutung haben, oder
R₂ ist der Rest (b) 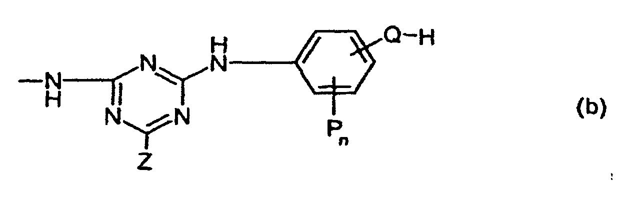 wobei
P für -SO₃H, -COOH oder -OH,
Q für einen Rest -NH- oder eine unter 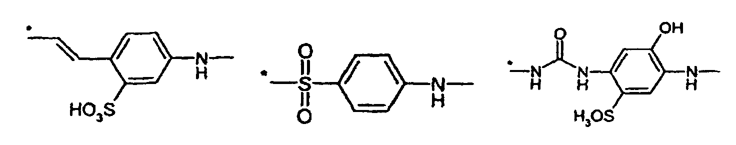 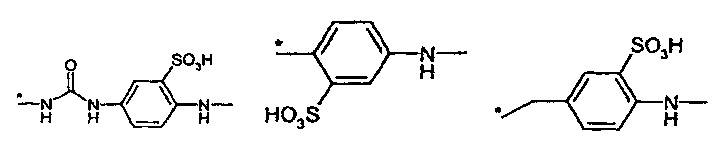 oder 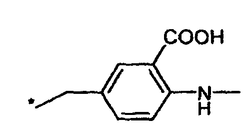 wobei der Stern jeweils die Bindung mit dem Phenylring markiert, ausgewählte Gruppe steht,
Z die obengenannte Bedeutung hat und
n den Wert 0, 1, 2 besitzt,
R₃ bedeutet -NR₆,
wobei R₆ für Wasserstoff oder einen C₁₋₄-Alkylrest steht,
oder
R₃ ist der Rest (a) 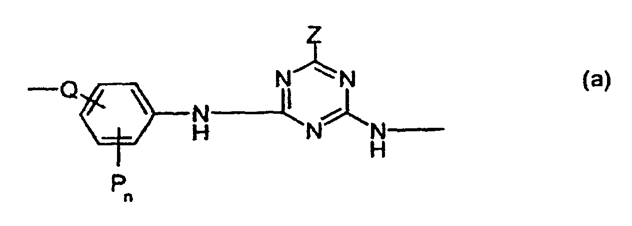 wobei alle Substituenten die obengenannte Bedeutung haben,
R₄ bedeutet Wasserstoff oder C₁₋₄-Alkyl,
T₁ und T₂ bedeuten unabhängig voneinander Wasserstoff, Halogen, C₁₋₆-Alkyl oder C₁₋₄-Alkoxy,
M bedeutet Wasserstoff oder ein Kation,
mit den Massgaben, dass
(i) bei R₁ und R₂ gleich -NH₂ R₃ für 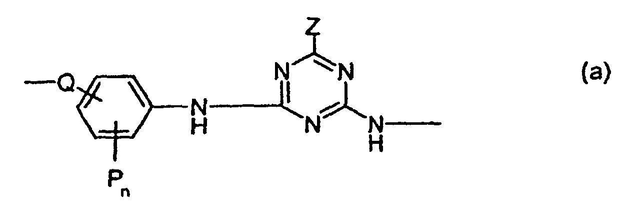 steht, wobei P, Q, Z und n die obengenannte Bedeutung haben,
(ii) bei R₁ gleich -NH₂ und R₂ gleich 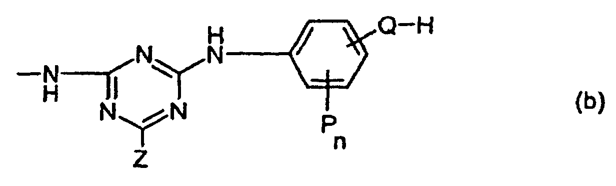 wobei P, Q, Z und n die obengenannte Bedeutung haben,
R₃ für -NH- steht,
deren Salze und Gemische derartiger Verbindungen und/oder Salze.

2. Verbindungen gemäss Anspruch 1, bei denen R₁ und R₂ gleichzeitig 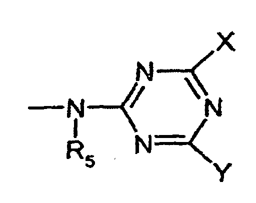 bedeuten, wobei
R₅ für Wasserstoff oder C₁₋₄-Alkyl steht,
und
R₃ -NR₆ bedeutet,
wobei R₆ für Wasserstoff oder einen C₁₋₄-Alkylrest steht,
deren Salze und Gemische derartiger Verbindungen und/oder Salze.

3. Verbindungen oder Gemische gemäss den Ansprüchen 1 bis 2, bei denen die Reste X, Y und Z keine chromophore Gruppe enthalten.

4. Verbindungen oder Gemische gemäss einem der Ansprüche 1 bis 3, bei denen ein Amin H-X, H-Y oder H-Z ein Molekulargewicht von 50 bis 500 und bevorzugt von 50 bis 400 besitzt.

5. Verbindungen oder Gemische gemäss einem der Ansprüche 1 bis 4, bei denen ein Amin H-X, H-Y oder H-Z keinen oder 1 bis 4 Ringe und bevorzugt keinen oder 1 bis 2 Ringe enthält.

6. Verbindungen oder Gemische gemäss einem der Ansprüche 1 bis 5, bei denen mindestens ein Aminrest X, Y und Z mindestens eine hydrophile Gruppe enthält.

7. Verbindungen oder Gemische gemäss Anspruch 6, bei denen hydrophile Gruppen oder Substituenten unabhängig voneinander anionisch, kationisch oder nichtionogen sind.

8. Verbindungen oder Gemische gemäss einem der Ansprüche 1 bis 7, **dadurch gekennzeichnet, dass** H-X und H-Y unabhängig voneinander: Ammoniak und aliphatische Amine, bevorzugt mit einer Hydroxy-, Carboxy- oder Sulfonsäuregruppe substituiert, wie z.B. Ethanolamin, Diethanolamin, Isopropanolamin, Diisopropanolamin, 2-Aminohydroxypropan, Glycin, N-Methylethanolamin, 3-Methoxypropylamin, 1-Aminoethyl-2-sulfonsäure und ganz besonders bevorzugt 1-Methylaminoethyl-2-sulfonsäure; aromatische Amine, bevorzugt mit einer Carboxy-, Alkoxy- und/oder Sulfonsäuregruppe substituiert, wie z.B. Anilin, 4-oder 3-Sulfoanilin, 4- oder 3-Carboxyanilin; und heterocyclische Amine, wie z.B. Morpholin, Piperazin oder Hydroxyethylpiperazin; oder N,N-Diethylaminopropylamin und 1,2-Diaminopropan bedeuten und H-Z die gleiche Bedeutung, ausgenommen die aromatische Amine, hat.

9. Verbindungen oder Gemische gemäss einem der Ansprüche 1 bis 8, bei der M Wasserstoff oder ein farbloses Kation ist oder M zur Bildung eines inneren Salzes ein kationischer Anteil an einem eine kationische Ladung enthaltenden Substituenten X, Y oder Z ist.

10. Gemische, enthaltend
a) eine Verbindung der Formel (la) gemäss Anspruch 1 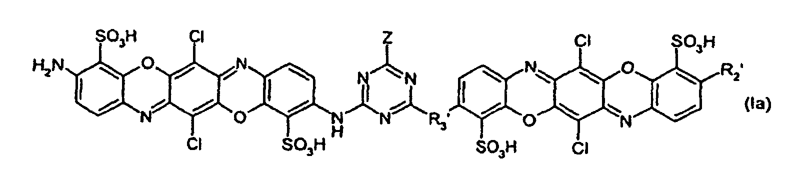 wobei R₃' einen zweiwertigen Rest der Formel (a) und R₂' -NH₂ bedeutet und Z die in Anspruch 1 angegebene Bedeutung hat.
b) eine Verbindung der obigen Formel (Ia), in der R₃' -NH- und R₂' einen Rest der Formel (b) bedeutet und Z die in Anspruch 1 angegebene Bedeutung hat.
c) ein Verbindung der Formel (IIc) eine Verbindung der Formel (II) 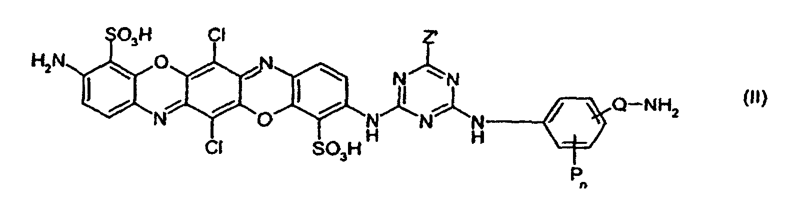 wobei Q, P und n die obengenannte Bedeutung haben und Z' neben der gleichen Bedeutung wie Z auch ein aromatisches Amin bedeuten kann, wobei Z' bevorzugt für die Komponente c) einen Rest der Formel (c) 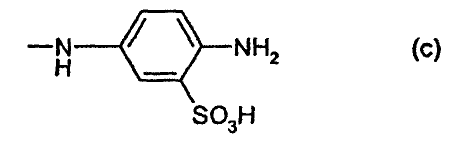 bedeutet,
d) eine Verbindung der Formel (II), in der Z' die obengenannte Bedeutung, ausgenommen Halogen, hat, wobei Z' bevorzugt einen Rest der Formel (d) 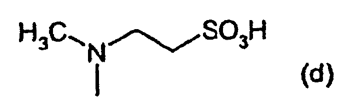 bedeutet,
e) eine Verbindung der Formel (III) 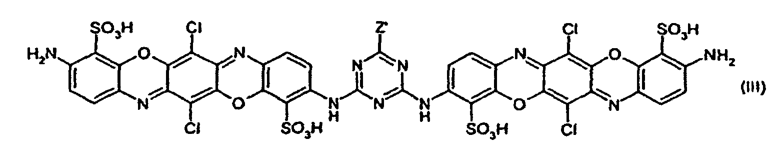 wobei und Z' neben der gleichen Bedeutung wie Z zusätzlich ein aromatisches Amin bedeuten kann,
oder Salze derartiger Verbindungen.

11. Gemische gemäss Anspruch 10, enthaltend in etwa
30 - 40% des Bestandteils a),
30 - 47% des Bestandteils b),
1,5 - 8% des Bestandteils c),
2 - 11% des Bestandteils d) sowie
12,5 - 23,5% des Bestandteils e)
in Form dessen Alkalimetallsalze.

12. Verfahren zur Herstellung einer Verbindung der Formel (Ib) 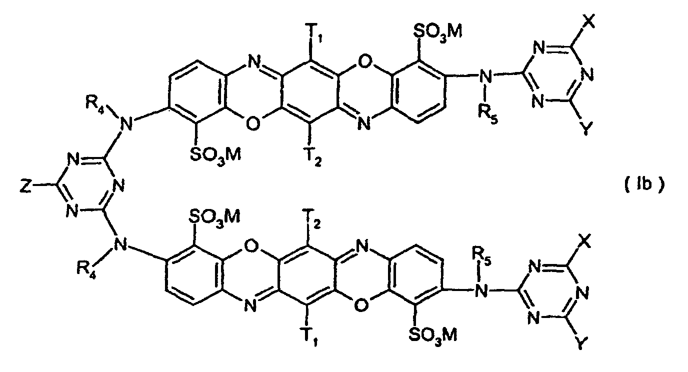 gemäss Anspruch 1 mit den gleichen Bedeutungen für die Substituenten wie in Anspruch 1,
bei dem man ein Mol einer Verbindung der Formel (IV) 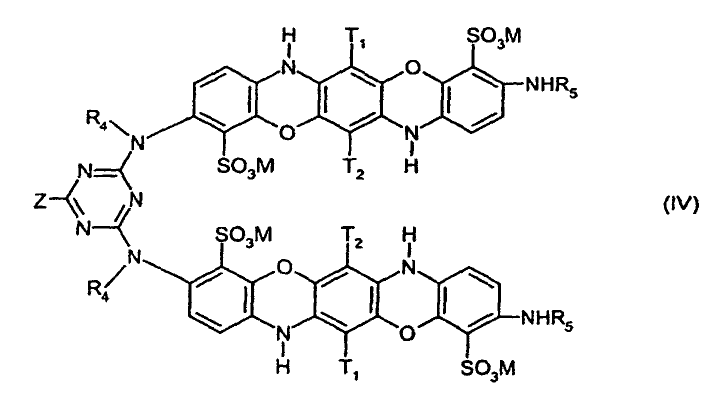 oder ein Gemisch von Verbindungen der Formel (IV) mit den gleichen Bedeutungen für die Substituenten wie in Formel (Ib) mit zwei Molen mindestens einer Verbindung der Formel (V) 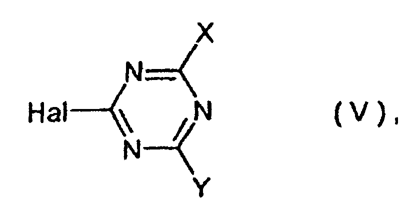 wobei Hal Halogen, bevorzugt Chlor, bedeutet und X und Y unabhängig voneinander die in Anspruch 1 genannte Bedeutung haben, dehydrohalogenierend umsetzt und, falls in der Formel (Ib) einer der Reste X oder Y eine von Halogen verschiedene Bedeutung hat, während in der Formel (V) der entsprechende Rest X bzw. Y Halogen bedeutet, und/oder falls in der Formel (Ib) Z eine von Halogen verschiedene Bedeutung hat, das erhaltene Kondensationsprodukt der Formel (VI) 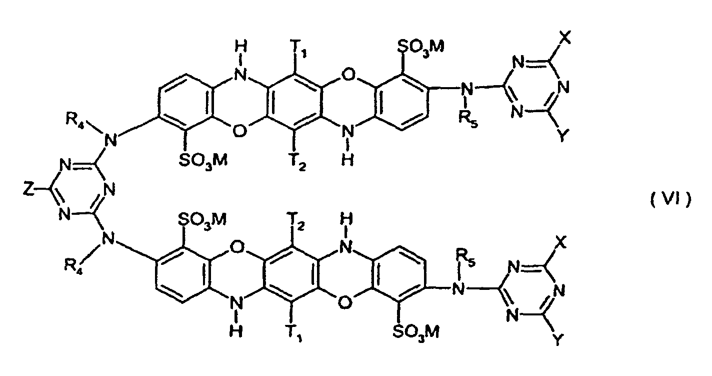 mit den gleichen Bedeutungen für die Substituenten wie in Formel (Ib) bei Bedarf vor Oxidation oder unter gleichzeitiger Oxidation mit mindestens einem entsprechenden Amin der Formel H-X, H-Y und/oder H-Z und/oder einem Alkalimetall C₁₋₃-Alkoholat oder Phenolat umsetzt.

13. Verfahren zur Herstellung von Verbindungen der Formel (la), deren Salze und Gemische derartiger Verbindungen und/oder Salze gemäss den Ansprüchen 1 bis 9, bei dem man eine Verbindung der Formel (VIII) 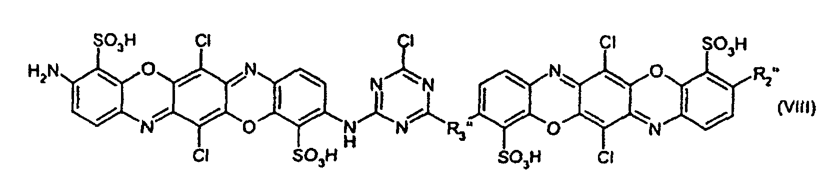 wobei entweder R₃" einen zweiwertigen Rest der Formel (a') 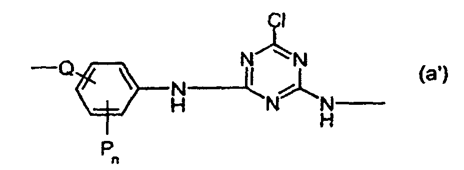 wobei P, Q und n die obengenannte Bedeutung haben
und R₂" -NH₂ oder
R₃" -NH- und R₂" einen Rest der Formel (b') 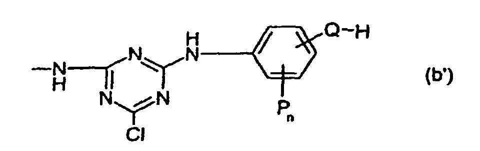 wobei P, Q und n die obengenannte Bedeutung haben, bedeutet,
oder ein Gemisch derartiger Verbindungen mit einem Amin H-Z oder H-Z' umsetzt und gewünschtenfalls so erhaltene Verbindungen der Formel (I) in Salze oder so erhaltene Salze in Verbindungen der Formel (I) oder in andere Salze überführt.

14. Verfahren zur Herstellung eines Gemischs gemäss Anspruch 10 oder Anspruch 11, bei dem man
a) eine Verbindung der Formel (IX) 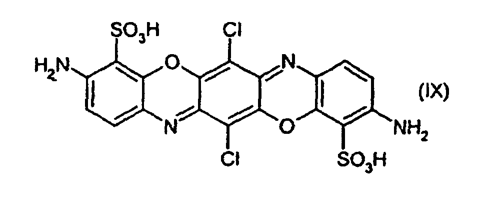 mit etwa einem Äquivalent 2,4,6-Trichlortriazin umsetzt,
b) die so erhaltene Verbindung der Formel (X) 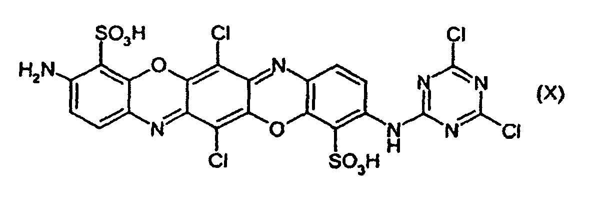 mit 1,4-Diaminobenzol-2-sulfonsäure umsetzt,
c) das so erhaltene Produkt mit einer weiteren Menge der Verbindung der obigen Formel (X) umsetzt,
d) das so erhaltene Produkt der Formel (VIII) mit einem Amin H-Z oder H-Z' umsetzt und
e) gewünschtenfalls das so erhaltene Gemisch von Verbindungen der Formeln (I), (II) und (III) in ein Gemisch von Salzen oder ein so erhaltenes Gemisch von Salzen in ein Gemisch von Verbindungen der Formeln (I), (II) und (III) oder in ein Gemisch von anderen Salzen überführt.

15. Gemische, erhalten nach dem Verfahren gemäss Anspruch 14.

16. Farbstoffzusammensetzung, enthaltend mindestens einen Farbstoff der Formel (I) gemäss einem der Ansprüche 1 bis 9 oder Salze davon oder ein Gemisch derartiger Verbindungen und/oder Salze oder ein Gemisch gemäss den Ansprüchen 10, 11, 14 oder 15.

17. Flüssige Farbstoffpräparation, enthaltend eine Zusammensetzung gemäss Anspruch 16, erhalten durch Ultrafiltration.

18. Verwendung einer Verbindung der Formel (I) gemäss einem der Ansprüche 1 bis 9 oder ein Gemisch gemäss den Ansprüchen 10 oder 11, gegebenenfalls in Form einer Zusammensetzung gemäss Anspruch 16, als Farbstoff zum Färben oder Bedrucken von Substraten oder als Komponente von Druckfarben.

19. Verwendung nach Anspruch 18, bei der das zu färbende oder bedruckende Substrat Papier oder papierbasierendes Material ist.

20. Substrat, gefärbt oder bedruckt gemäss Anspruch 18.

21. Substrat nach Anspruch 20, bei dem es sich bei dem gefärbten oder bedruckten Substrat um Papier oder papierbasierendes Material handelt.

22. Verfahren zur Herstellung von Tintenstrahldrucktinten, **dadurch gekennzeichnet, dass** man eine Verbindung der Ansprüche 1 bis 9 oder deren Salze oder ein Gemisch derartiger Verbindungen und/oder Salze oder ein Gemisch gemäss den Ansprüchen 10, 11, 14 oder 15 einsetzt.

23. Tintenstrahldrucktinten, hergestellt nach dem Verfahren gemäss Anspruch 12.

## Revendications

1. Composés de la formula (I) 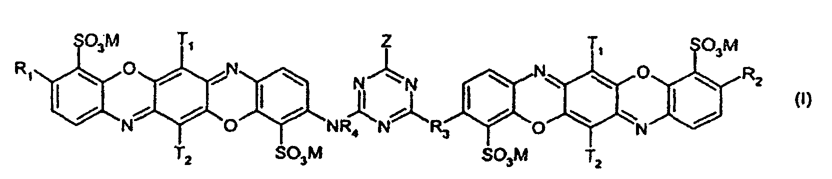 dans laquelle
R₁ signifie -NH₂ ou 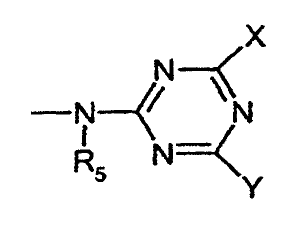 dans laquelle
R₅ signifie hydrogène ou alkyle en C₁₋₄,
X et Y signifient indépendamment halogène ou hydroxy ou alcoxy en C₁₋₃ ou phénoxy ou le radical d'une amine cyclique, aliphatique, araliphatique ou aromatique lié sur l'azote aminé et éventuellement substitué par des groupements hydroxy, carboxy, alcoxy, alkyle et/ou acide sulfonique ou le reste d'une amine hétéro-cyclique lié sur l'azote aminé,
Z signifie un radical de formule 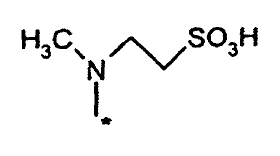 dans laquelle l'astérisque indique le point de fixation,
R₂ signifie -NH₂ ou 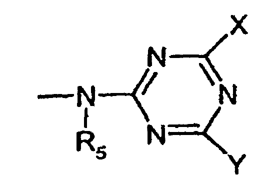 avec les mêmes définitions pour R₅, X et Y que celles ci-dessus, ou
R₂ est le reste (b) 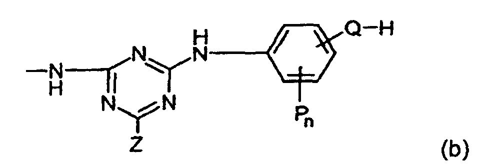 dans laquelle
P signifie -SO₃H, -COOH ou -OH,
Q signifie un radical -NH- ou un groupement choisi parmi 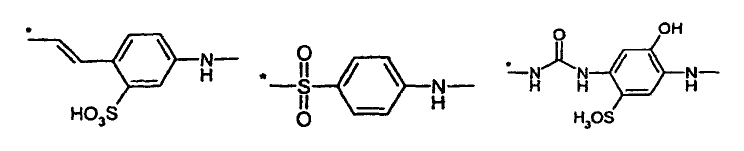 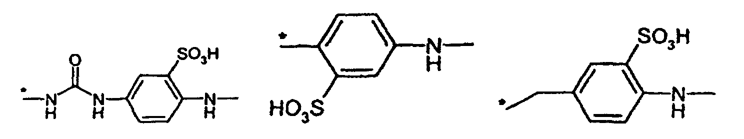 ou 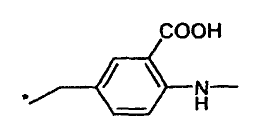 dans lesquels l'astérisque indique la liaison fixée au cycle phényle,
Z a la même signification que ci-dessus,
n prend la valeur 0, 1, 2,
R₃ signifie -NR₆
dans lequel R₆ signifie hydrogène ou un radical alkyle en C₁₋₄
ou
R₃ est le reste (a) 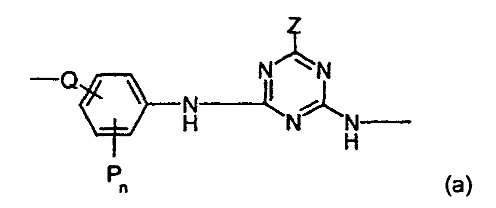 dans lequel tous les substituants ont les mêmes significations que celles définies ci-dessus,
R₄ signifie hydrogène ou alkyle en C₁₋₄,
T₁ et T₂ indépendamment l'un de l'autre, signifient hydrogène, halogène, alkyle en C₁₋₆ ou alcoxy en C₁₋₄,
M signifie hydrogène ou un cation;
sous réserve que
(i) lorsque R₁ et R₂ sont -NH₂, alors R₃ est 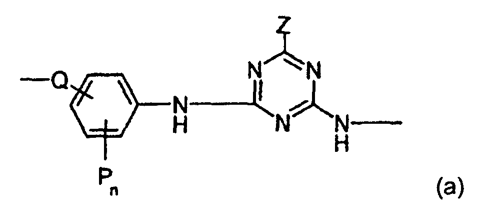 dans laquelle P, Q, Z et n ont les mêmes significations que celles ci-dessus,
(ii) lorsque R₁ est -NH₂ et R₂ est 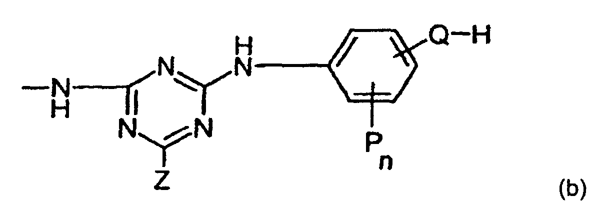 dans laquelle P, Q, Z et n ont les mêmes significations que celles ci-dessus,
alors R₃ est -NH-;
les sels de ceux-ci et les mélanges de tels composés et/ou sels.

2. Composé selon la revendication 1, dans lequel R₁ et R₂ signifient tous les deux 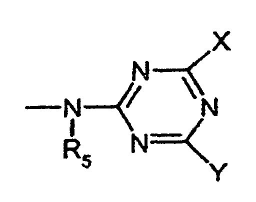 dans laquelle
R₅ signifie hydrogène ou alkyle en C₁₋₄,
et
R₃ signifie -NR₆
dans lequel R₆ signifie hydrogène ou un radical alkyle en C₁₋₄,
les sels de ceux-ci et les mélanges de tels composés et/ou sels.

3. Composé ou mélange selon les revendications 1 à 2, dans lequel les radicaux X, Y et Z ne contiennent aucun groupement chromophore.

4. Composé ou mélange selon l'une quelconque des revendications 1 à 3, dans lequel toute amine H-X, H-Y ou H-Z a un poids moléculaire dans la plage de 50 à 500, préférablement de 50 à 400.

5. Composé ou mélange selon l'une quelconque des revendications 1 à 4, dans lequel toute amine H-X, H-Y ou H-Z ne contient aucun cycle ou contient de 1 à 4 cycles, de préférence aucun cycle ou de 1 à 2 cycles.

6. Composé ou mélange selon l'une quelconque des revendications 1 à 5, dans lequel au moins un radical amine X, Y et Z contient au moins un groupement hydrophile.

7. Composé ou mélange selon la revendication 6, dans lequel chaque groupement ou substituant hydrophile est indépendamment anionique, cationique ou non ionogène.

8. Composé ou mélange selon l'une quelconque des revendications 1 à 7, caractérisé en ce que H-X et H-Y sont indépendamment l'ammoniac et les amines aliphatiques, substituées de préférence par un groupement hydroxy, carboxy ou acide sulfonique, par exemple l'éthanolamine, la diéthanolamine, l'isopropanolamine, la diisopropanolamine, le 2-aminohydroxypropane, la glycine, la N-méthyléthanolamine, la 3-méthoxypropylamine, l'acide I-aminoéthyl-2-sulfonique et tout préférablement, l'acide 1-méthylaminoéthyl-2-sulfonique; des amines aromatiques substituées de préférence par un groupement carboxy, alcoxy et/ou acide sulfonique, par exemple l'aniline, la 4- ou 3-sulfoaniline et la 4- ou 3-carboxyaniline; et les amines hétérocycliques, par exemple la morpholine, la pipérazine ou l'hydroxyéthylpipérazine; ou la N,N-diéthylaminopropylamine et le 1,2-diaminopropane, et H-Z a les mêmes significations, à l'exception des amines aromatiques.

9. Composé ou mélange selon l'une quelconque des revendications 1 à 8, dans lequel M est hydrogène ou un cation incolore ou M est une portion cationique dans un substituant X, Y ou Z contenant une charge cationique, pour former un sel interne.

10. Mélanges contenant
a) un composé de formule (la) selon la revendication 1 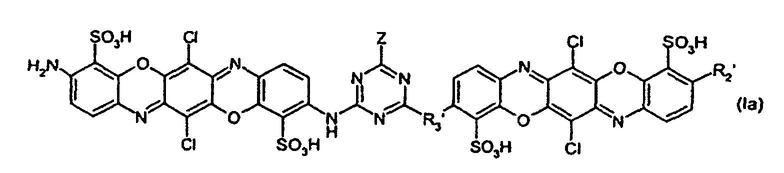 dans laquelle R₃' est un radical divalent de formule (a), R₂' est -NH₂ et Z a les mêmes significations que celles définies selon la revendication 1;
b) un composé de formule (la) ci-dessus dans laquelle R₃' est -NH-, R₂' est un radical de formule (b) et Z a les mêmes significations que celles définies selon la revendication 1;
c) un composé de formule (II) 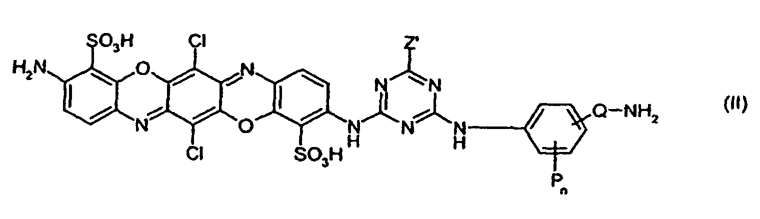 dans laquelle Q, P et n ont la même signification que celle définie ci-dessus et Z' a la même définition que Z, en outre Z' peut être une amine aromatique, de préférence pour le composant c) Z' est un radical de formule (c) 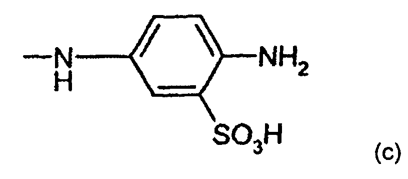
d) un composé de formule (II), dans laquelle Z' a les mêmes significations que celles définies ci-dessus, à l'exception d'halogène, de préférence Z' est un radical de formule (d) 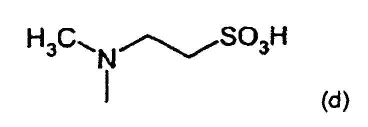
e) un composé de formule (III) 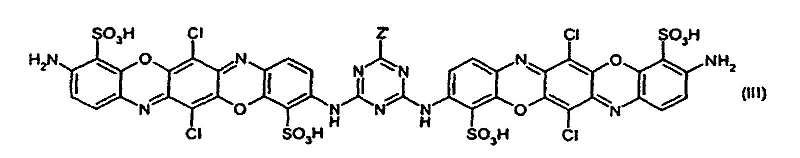 dans laquelle Z' a la même définition que Z, en outre Z' peut être une amine aromatique,
ou les sels de tels composés.

11. Mélanges selon la revendication 10, contenant approximativement
30-40% de composant a),
30-47% de composant b),
1,5-8% de composant c),
2-11 % de composant d), et
12,5-23,5% de composant e)
sous forme de ses sels de métaux alcalins.

12. Procédé de préparation d'un composé de formule (Ib) 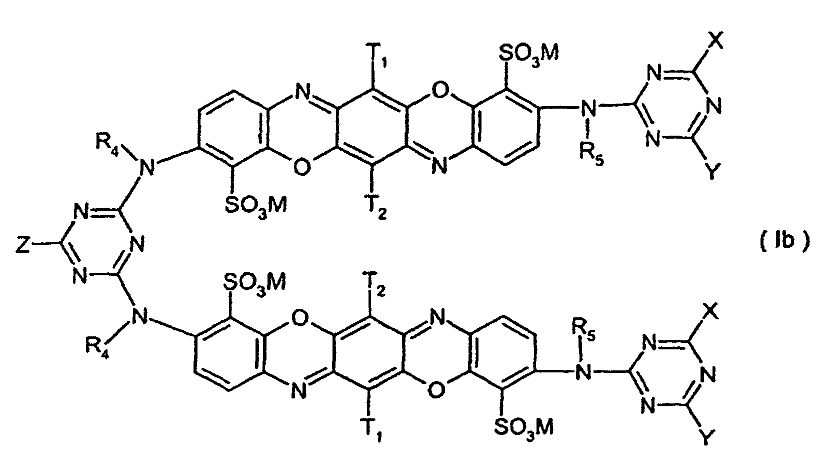 selon la revendication 1, ayant les mêmes définitions des substituants que celles selon la revendication 1,
dans lequel une mole d'un composé de formule (IV) 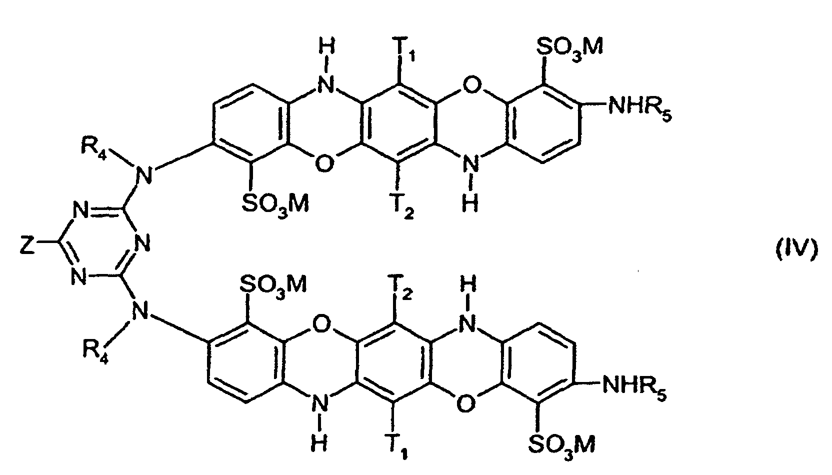 ou un mélange de composés de formule (IV) ayant les mêmes définitions des substituants que celles dans la formule (Ib), est réagie avec deux moles d'au moins un composé de formule (V) 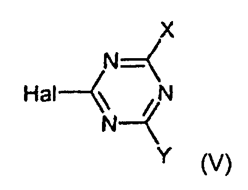 dans laquelle Hal signifie halogène, de préférence chlore, et X et Y ont indépendamment les mêmes significations que celles définies selon la revendication 1, dans des conditions de déshydrohalogénation, et si, dans la formule (Ib), l'un quelconque parmi X et Y a une signification autre qu'halogène, alors que dans la formule (V) le X ou Y correspondant signifie halogène, et/ou si, dans la formule (Ib), Z a une signification autre qu'halogène, le produit de condensation de formule (VI) obtenu 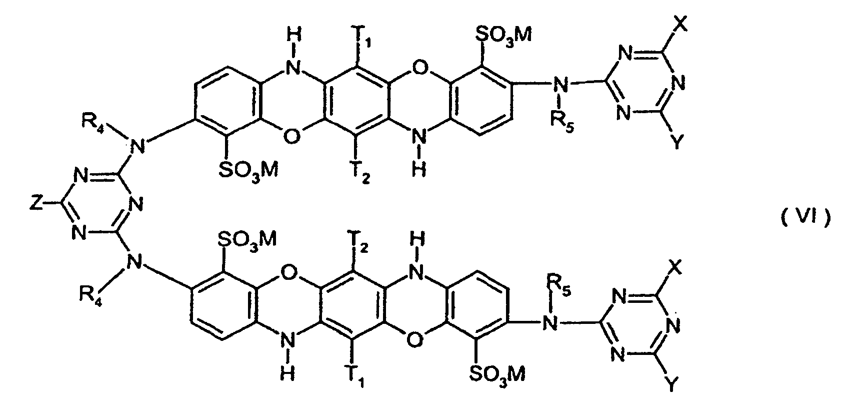 ayant les mêmes définitions des substituants que celles dans la formule (Ib), est réagi, avant l'oxydation ou simultanément avec l'oxydation, avec au moins une amine correspondante de formule H-X, H-Y et/ou H-Z et/ou un alcoolate en C₁₋₃ ou phénolate de métal alcalin, le cas échéant.

13. Procédé de préparation de composés de formule (la), de sels de ceux-ci et de mélanges de tels composés et/ou sels selon les revendications 1 à 9, comprenant la réaction d'un composé de formule (VIII) 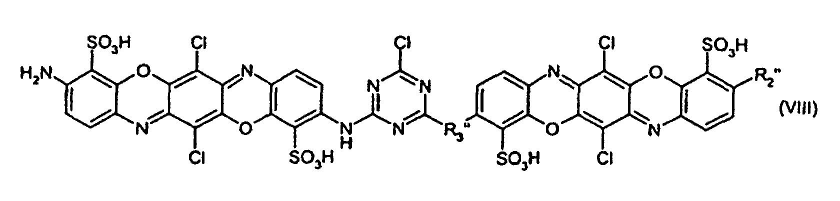 dans laquelle soit R₃" est un radical divalent de formule (a') 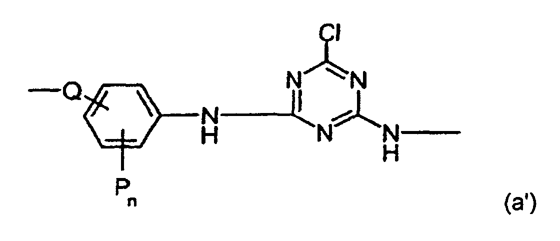 dans laquelle P, Q et n ont les mêmes significations que celles définies ci-dessus et R₂" est -NH₂; soit
R₃" est -NH- et R₂" est un radical de formule (b') 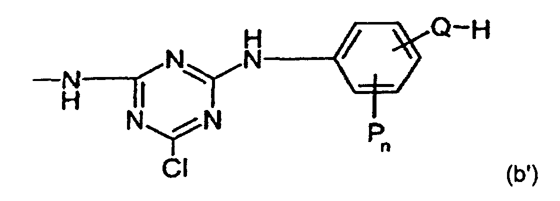 dans laquelle P, Q et n ont les mêmes significations que celles définies ci-dessus
ou un mélange de tels composés, avec une amine H-Z ou H-Z' et, si on le désire, la conversion des composés de formule (I) ainsi obtenus en sels, ou des sels ainsi obtenus en composés de formule (I) ou en d'autres sels.

14. Procédé de préparation d'un mélange selon la revendication 10 ou la revendication 11, comprenant
a) la réaction d'un composé de formule (IX) 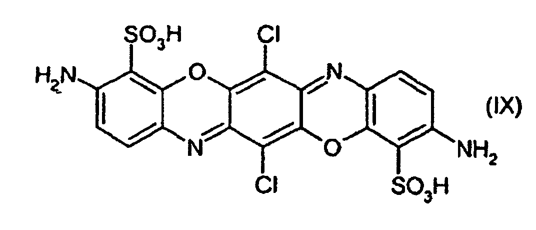 avec environ un équivalent de 2,4,6-trichlorotriazine,
b) la réaction du composé de formule (X) 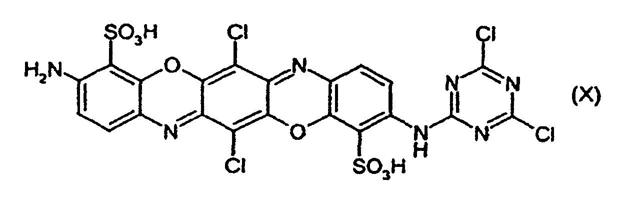 ainsi obtenu avec de l'acide 1,4-diaminobenzène-2-sulfonique;
c) la réaction du produit ainsi obtenu avec une autre portion du composé de formule (X) ci-dessus;
d) la réaction du composé de formule (VIII) ainsi obtenu avec une amine H-Z ou H-Z'; et
e) si on le désire, la conversion du mélange de composés de formules (I), (II) et (III) ainsi obtenu en un mélange de sels, ou la conversion d'un mélange de sels ainsi obtenu en un mélange de composés de formules (I), (II) et (III) ou en un mélange d'autres sels.

15. Mélanges obtenus par le procédé selon la revendication 14.

16. Composition de teinture comprenant au moins un colorant de formule (I) selon l'une quelconque des revendications 1 à 9 ou des sels de ceux-ci ou un mélange de tels composés et/ou sels, ou un mélange selon les revendications 10, 11, 14 ou 15.

17. Préparation de teinture liquide contenant une composition selon la revendication 16, obtenue par ultrafiltration.

18. Utilisation d'un composé de formule (I) selon l'une quelconque des revendications 1 à 9 ou d'un mélange selon les revendications 10 ou 11, éventuellement sous la forme d'une composition selon la revendication 16, comme colorant pour la teinture ou l'impression de substrats ou comme composant d'encres d'imprimerie.

19. Utilisation selon la revendication 18, dans laquelle le substrat à colorer ou à imprimer est du papier ou un matériau à base de papier.

20. Substrat coloré ou imprimé selon la revendication 18.

21. Substrat selon la revendication 20, dans lequel le substrat ayant été coloré ou imprimé est du papier ou un matériau à base de papier.

22. Procédé de préparation d'encres pour jets d'encre, **caractérisé en ce qu'**un composé selon les revendications 1 à 9 ou des sels de ceux-ci ou un mélange de tels composés et/ou sels, ou un mélange selon les revendications 10, 11, 14 ou 15, est utilisé.

23. Encres pour jets d'encre préparées par le procédé selon la revendication 12.
